# EUROPEAN PATENT APPLICATION

(11) **EP 2 128 252 A1**
(43) Date of publication of application: **02.12.2009**
(21) Application number: 08711133.2
(22) Date of filing: 12.02.2008
(51) Int. Cl.: C12N 15/09, C07K 19/00, C12N 1/21, C12P 21/02, C12P 21/06

(54) **FUSED PROTEIN CONTAINING INSULIN PRECURSOR OF OVEREXPRESSION AND SECRETION TYPE, DNA ENCODING THE SAME AND METHOD OF PRODUCING INSULIN**

(71) Applicant: Itoham Foods Inc., Kobe-shi, Hyogo 657-0037 (JP); Udaka, Shigezo, Nagoya-shi, Aishi 464-0808 (JP)
(72) Inventor: SATO, Seiji, - (JP); KONDO, Masa-aki, Moriya-shi, Ibaraki 302-0128 (JP); KUDO, Toshiyuki, - (JP); ENDO, Ko-suke, - (JP)
(74) Representative: Holliday, Louise Caroline
(86) International application number: PCT/JP2008/052275
(87) International publication number: WO 2009/101672

(57) **Abstract**

This invention relates to DNA encoding a fusion protein comprising highly-expressed and -secreted insulin precursors for producing recombinant insulins, and to a method for producing insulin using such DNA.

SEQUENCE LISTING

<110>  ITOHAM FOODS Inc.
       SHIGEZO UDAKA

<120>  Fusion protein containing highly-expressed and -secreted insulin
       precursor, DNA encoding same, and method of producing insulin

<130>  P034590EP

<140>  PCT/JP2008/052275
<141>  2008-02-12

<160>  52

<170>  PatentIn version 3.4

<210>  1
<211>  5
<212>  PRT
<213>  Artificial

<220>
<223>  linker

<400>  1

Asp Gly Asp Arg Arg
1               5


<210>  2
<211>  5
<212>  PRT
<213>  Artificial

<220>
<223>  linker

<400>  2

Leu Asn Ser Ala Arg
1               5


<210>  3
<211>  4
<212>  PRT
<213>  Artificial

<220>
<223>  linker

<400>  3

Gly Ser Pro Arg
1


<210>  4
<211>  5
<212>  PRT
<213>  Artificial

<220>
<223>  linker

<400>  4

Asp Leu Asp Arg Arg
1               5


<210>  5
<211>  5
<212>  PRT
<213>  Artificial

<220>
<223>  linker

<400>  5

Asp Asn Asp Arg Arg
1               5


<210>  6
<211>  4
<212>  PRT
<213>  Artificial

<220>
<223>  linker

<400>  6

Asp Gly Asp Arg
1


<210>  7
<211>  5
<212>  PRT
<213>  Artificial

<220>
<223>  linker

<400>  7

Arg Asp Gly Asp Arg
1               5


<210>  8
<211>  50
<212>  PRT
<213>  Artificial

<220>
<223>  insulin precursor

<400>  8

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Gly Ile Val
            20                  25                  30

Glu Gln Cys Cys Thr Ser Ile Cys Ser Leu Tyr Gln Leu Glu Asn Tyr
        35                  40                  45

Cys Asn
    50

<210>  9
<211>  50
<212>  PRT
<213>  Artificial

<220>
<223>  insulin precursor

<400>  9

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Gly Ile Val
            20                  25                  30

Glu Gln Cys Cys Thr Ser Ile Cys Ser Leu Tyr Gln Leu Glu Asn Tyr
        35                  40                  45

Cys Asn
    50

<210>  10
<211>  80
<212>  PRT
<213>  Artificial

<220>
<223>  fusion protein

<400>  10

Val Val Asn Ser Val Leu Ala Ser Ala Leu Ala Leu Thr Val Ala Pro
1               5                   10                  15

Met Ala Phe Ala Ala Glu Glu Ala Ala Asp Gly Asp Arg Arg Phe Val
            20                  25                  30

Asn Gln His Leu Cys Gly Ser His Leu Val Glu Ala Leu Tyr Leu Val
        35                  40                  45

Cys Gly Glu Arg Gly Phe Phe Tyr Thr Pro Lys Gly Ile Val Glu Gln
    50                  55                  60

Cys Cys Thr Ser Ile Cys Ser Leu Tyr Gln Leu Glu Asn Tyr Cys Asn
65                  70                  75                  80

<210>  11
<211>  81
<212>  PRT
<213>  Artificial

<220>
<223>  fusion protein

<400>  11

Val Val Asn Ser Val Leu Ala Ser Ala Leu Ala Leu Thr Val Ala Pro
1               5                   10                  15

Met Ala Phe Ala Ala Glu Glu Ala Ala Thr Asp Gly Asp Arg Arg Phe
            20                  25                  30

Val Asn Gln His Leu Cys Gly Ser His Leu Val Glu Ala Leu Tyr Leu
        35                  40                  45

Val Cys Gly Glu Arg Gly Phe Phe Tyr Thr Pro Lys Gly Ile Val Glu
    50                  55                  60

Gln Cys Cys Thr Ser Ile Cys Ser Leu Tyr Gln Leu Glu Asn Tyr Cys
65                  70                  75                  80

Asn

<210>  12
<211>  81
<212>  PRT
<213>  Artificial

<220>
<223>  fusion protein

<400>  12

Val Val Asn Ser Val Leu Ala Ser Ala Leu Ala Leu Thr Val Ala Pro
1               5                   10                  15

Met Ala Phe Ala Ala Glu Glu Ala Ala Thr Leu Asn Ser Ala Arg Phe
            20                  25                  30

Val Asn Gln His Leu Cys Gly Ser His Leu Val Glu Ala Leu Tyr Leu
        35                  40                  45

Val Cys Gly Glu Arg Gly Phe Phe Tyr Thr Pro Lys Gly Ile Val Glu
    50                  55                  60

Gln Cys Cys Thr Ser Ile Cys Ser Leu Tyr Gln Leu Glu Asn Tyr Cys
65                  70                  75                  80

Asn

<210>  13
<211>  80
<212>  PRT
<213>  Artificial

<220>
<223>  fusion protein

<400>  13

Val Val Asn Ser Val Leu Ala Ser Ala Leu Ala Leu Thr Val Ala Pro
1               5                   10                  15

Met Ala Phe Ala Ala Glu Glu Ala Ala Thr Gly Ser Pro Arg Phe Val
            20                  25                  30

Asn Gln His Leu Cys Gly Ser His Leu Val Glu Ala Leu Tyr Leu Val
        35                  40                  45

Cys Gly Glu Arg Gly Phe Phe Tyr Thr Pro Lys Gly Ile Val Glu Gln
    50                  55                  60

Cys Cys Thr Ser Ile Cys Ser Leu Tyr Gln Leu Glu Asn Tyr Cys Asn
65                  70                  75                  80

<210>  14
<211>  82
<212>  PRT
<213>  Artificial

<220>
<223>  fusion protein

<400>  14

Val Val Asn Ser Val Leu Ala Ser Ala Leu Ala Leu Thr Val Ala Pro
1               5                   10                  15

Met Ala Phe Ala Ala Glu Glu Ala Ala Thr Thr Asp Gly Asp Arg Arg
            20                  25                  30

Phe Val Asn Gln His Leu Cys Gly Ser His Leu Val Glu Ala Leu Tyr
        35                  40                  45

Leu Val Cys Gly Glu Arg Gly Phe Phe Tyr Thr Pro Lys Gly Ile Val
    50                  55                  60

Glu Gln Cys Cys Thr Ser Ile Cys Ser Leu Tyr Gln Leu Glu Asn Tyr
65                  70                  75                  80

Cys Asn

<210>  15
<211>  82
<212>  PRT
<213>  Artificial

<220>
<223>  fusion protein

<400>  15

Val Val Asn Ser Val Leu Ala Ser Ala Leu Ala Leu Thr Val Ala Pro
1               5                   10                  15

Met Ala Phe Ala Ala Glu Glu Ala Ala Thr Thr Asp Leu Asp Arg Arg
            20                  25                  30

Phe Val Asn Gln His Leu Cys Gly Ser His Leu Val Glu Ala Leu Tyr
        35                  40                  45

Leu Val Cys Gly Glu Arg Gly Phe Phe Tyr Thr Pro Lys Gly Ile Val
    50                  55                  60

Glu Gln Cys Cys Thr Ser Ile Cys Ser Leu Tyr Gln Leu Glu Asn Tyr
65                  70                  75                  80

Cys Asn

<210>  16
<211>  82
<212>  PRT
<213>  Artificial

<220>
<223>  fusion protein

<400>  16

Val Val Asn Ser Val Leu Ala Ser Ala Leu Ala Leu Thr Val Ala Pro
1               5                   10                  15

Met Ala Phe Ala Ala Glu Glu Ala Ala Thr Thr Asp Asn Asp Arg Arg
            20                  25                  30

Phe Val Asn Gln His Leu Cys Gly Ser His Leu Val Glu Ala Leu Tyr
        35                  40                  45

Leu Val Cys Gly Glu Arg Gly Phe Phe Tyr Thr Pro Lys Gly Ile Val
    50                  55                  60

Glu Gln Cys Cys Thr Ser Ile Cys Ser Leu Tyr Gln Leu Glu Asn Tyr
65                  70                  75                  80

Cys Asn

<210>  17
<211>  87
<212>  PRT
<213>  Artificial

<220>
<223>  fusion protein

<400>  17

Val Val Asn Ser Val Leu Ala Ser Ala Leu Ala Leu Thr Val Ala Pro
1               5                   10                  15

Met Ala Phe Ala Ala Glu Glu Ala Ala Thr Thr Thr Ala Pro Lys Met
            20                  25                  30

Asp Gly Asp Arg Arg Phe Val Asn Gln His Leu Cys Gly Ser His Leu
        35                  40                  45

Val Glu Ala Leu Tyr Leu Val Cys Gly Glu Arg Gly Phe Phe Tyr Thr
    50                  55                  60

Pro Lys Gly Ile Val Glu Gln Cys Cys Thr Ser Ile Cys Ser Leu Tyr
65                  70                  75                  80

Gln Leu Glu Asn Tyr Cys Asn
                85

<210>  18
<211>  81
<212>  PRT
<213>  Artificial

<220>
<223>  fusion protein

<400>  18

Val Val Asn Ser Val Leu Ala Ser Ala Leu Ala Leu Thr Val Ala Pro
1               5                   10                  15

Met Ala Phe Ala Ala Glu Glu Ala Ala Thr Thr Asp Gly Asp Arg Phe
            20                  25                  30

Val Asn Gln His Leu Cys Gly Ser His Leu Val Glu Ala Leu Tyr Leu
        35                  40                  45

Val Cys Gly Glu Arg Gly Phe Phe Tyr Thr Pro Lys Gly Ile Val Glu
    50                  55                  60

Gln Cys Cys Thr Ser Ile Cys Ser Leu Tyr Gln Leu Glu Asn Tyr Cys
65                  70                  75                  80

Asn

<210> 19
<211> 240
<212> DNA
<213> Artificial

<220>
<223> fusion protein

<400> 19
gtcgttaaca gtgtattggc tagtgcactc gcacttactg ttgctccaat ggctttcgca 60

gcagaagaag cagcagacgg tgatcgtcgc tttgtgaacc aacacctgtg cggctcacac 120

ctggtggaag ctctctacct agtgtgcggg gaaagaggct tcttctacac acccaagggc 180

attgtggaac aatgctgtac cagcatctgc tccctctacc agctggagaa ctactgcaac 240

<210> 20
<211> 243
<212> DNA
<213> Artificial

<220>
<223> fusion protein

<400> 20
gtcgttaaca gtgtattggc tagtgcactc gcacttactg ttgctccaat ggctttcgca 60

gcagaagaag cagcaactga cggtgatcgt cgctttgtga accaacacct gtgcggctca 120

cacctggtgg aagctctcta cctagtgtgc ggggaaagag gcttcttcta cacacccaag 180

ggcattgtgg aacaatgctg taccagcatc tgctccctct accagctgga gaactactgc 240

aac 243

<210> 21
<211> 243
<212> DNA
<213> Artificial

<220>
<223> fusion protein

<400> 21

gtcgttaaca gtgtattggc tagtgcactc gcacttactg ttgctccaat ggctttcgca 60

gcagaagaag cagcaactct gaacagcgct cgctttgtga accaacacct gtgcggctca 120

cacctggtgg aagctctcta cctagtgtgc ggggaaagag gcttcttcta cacacccaag 180

ggcattgtgg aacaatgctg taccagcatc tgctccctct accagctgga gaactactgc 240

aac 243

<210> 22
<211> 240
<212> DNA
<213> Artificial

<220>
<223> fusion protein

<400> 22
gtcgttaaca gtgtattggc tagtgcactc gcacttactg ttgctccaat ggctttcgca 60

gcagaagaag cagcaactgg ttctcctcgc tttgtgaacc aacacctgtg cggctcacac 120

ctggtggaag ctctctacct agtgtgcggg gaaagaggct tcttctacac acccaagggc 180

attgtggaac aatgctgtac cagcatctgc tccctctacc agctggagaa ctactgcaac 240

<210> 23
<211> 246
<212> DNA
<213> Artificial

<220>
<223> fusion protein

<400> 23
gtcgttaaca gtgtattggc tagtgcactc gcacttactg ttgctccaat ggctttcgca 60

gcagaagaag cagcaactac tgacggtgat cgtcgctttg tgaaccaaca cctgtgcggc 120

tcacacctgg tggaagctct ctacctagtg tgcggggaaa gaggcttctt ctacacaccc 180

aagggcattg tggaacaatg ctgtaccagc atctgctccc tctaccagct ggagaactac 240

tgcaac 246

<210> 24
<211> 246
<212> DNA
<213> Artificial

<220>
<223> fusion protein

<400> 24
gtcgttaaca gtgtattggc tagtgcactc gcacttactg ttgctccaat ggctttcgca 60

gcagaagaag cagcaactac tgacttggat cgtcgctttg tgaaccaaca cctgtgcggc 120

tcacacctgg tggaagctct ctacctagtg tgcggggaaa gaggcttctt ctacacaccc 180

aagggcattg tggaacaatg ctgtaccagc atctgctccc tctaccagct ggagaactac 240

tgcaac 246

<210> 25
<211> 246
<212> DNA
<213> Artificial

<220>
<223> fusion protein

<400> 25
gtcgttaaca gtgtattggc tagtgcactc gcacttactg ttgctccaat ggctttcgca 60

gcagaagaag cagcaactac tgacaatgat cgtcgctttg tgaaccaaca cctgtgcggc 120

tcacacctgg tggaagctct ctacctagtg tgcggggaaa gaggcttctt ctacacaccc 180

aagggcattg tggaacaatg ctgtaccagc atctgctccc tctaccagct ggagaactac 240

tgcaac 246

<210> 26
<211> 261
<212> DNA
<213> Artificial

<220>
<223> fusion protein

<400> 26
gtcgttaaca gtgtattggc tagtgcactc gcacttactg ttgctccaat ggctttcgca 60

gcagaagaag cagcaactac tacagctcca aaaatggacg gtgatcgtcg ctttgtgaac 120

caacacctgt gcggctcaca cctggtggaa gctctctacc tagtgtgcgg ggaaagaggc 180

ttcttctaca cacccaaggg cattgtggaa caatgctgta ccagcatctg ctccctctac 240

cagctggaga actactgcaa c 261

<210> 27
<211> 261
<212> DNA
<213> Artificial

<220>
<223> fusion protein

<400> 27
gtcgttaaca gtgtattggc tagtgcactc gcacttactg ttgctccaat ggctttcgca 60

gcagaagaag cagcaactac tgacggtgat cgctttgtga accaacacct gtgcggctca 120

cacctggtgg aagctctcta cctagtgtgc ggggaaagag gcttcttcta cacacccaag 180

acccgcgatg gcgaccgtgg cattgtggaa caatgctgta ccagcatctg ctccctctac 240

cagctggaga actactgcaa c 261

<210>  28
<211>  12
<212>  PRT
<213>  Artificial

<220>
<223>  leader peptide

<400>  28

Ala Glu Glu Ala Ala Thr Thr Thr Ala Pro Lys Met
1               5                   10


<210>  29
<211>  12
<212>  PRT
<213>  Artificial

<220>
<223>  leader peptide

<400>  29

Ala Pro Lys Asp Gly Ile Tyr Ile Gly Gly Asn Ile
1               5                   10


<210>  30
<211>  12
<212>  PRT
<213>  Artificial

<220>
<223>  leader peptide

<400>  30

Ala Glu Asp Thr Thr Thr Ala Pro Lys Met Asp Ala
1               5                   10


<210>  31
<211>  20
<212>  DNA
<213>  Artificial

<220>
<223>  primer

<400>  31
acacgcgctt gcaggattcg                                                   20


<210>  32
<211>  18
<212>  DNA
<213>  Artificial

<220>
<223>  primer

<400>  32

tgctgcttct tctgctgc                                                    18

<210>  33
<211>  18
<212>  DNA
<213>  Artificial

<220>
<223>  primer

<400>  33
agttgctgct tcttctgc                                                    18

<210>  34
<211>  18
<212>  DNA
<213>  Artificial

<220>
<223>  primer

<400>  34
agtagttgct gcttcttc                                                    18

<210>  35
<211>  18
<212>  DNA
<213>  Artificial

<220>
<223>  primer

<400>  35
catttttgga gctgtagt                                                    18

<210>  36
<211>  19
<212>  DNA
<213>  Artificial

<220>
<223>  primer

<400>  36
atggccctgt ggatgcgcc                                                   19

<210>  37
<211>  19
<212>  DNA
<213>  Artificial

<220>
<223>  primer

<400>  37
ctagttgcag tagttctcc                                                   19

<210>  38

Page 12

<211>  18
<212>  DNA
<213>  Artificial

<220>
<223>  primer

<400>  38
tttgtgaacc aacacctg                                                    18


<210>  39
<211>  30
<212>  DNA
<213>  Artificial

<220>
<223>  primer

<400>  39
gacggtgatc gctttgtgaa ccaacacctg                                       30


<210>  40
<211>  21
<212>  DNA
<213>  Artificial

<220>
<223>  primer

<400>  40
gcgggtcttg ggtgtgtaga a                                                21


<210>  41
<211>  30
<212>  DNA
<213>  Artificial

<220>
<223>  primer

<400>  41
gacggtgatc gtcgctttgt gaaccaacac                                       30


<210>  42
<211>  18
<212>  DNA
<213>  Artificial

<220>
<223>  primer

<400>  42
cttgggtgtg tagaagaa                                                    18


<210>  43
<211>  33
<212>  DNA
<213>  Artificial

<220>
<223>  primer

<400>  43
gacttggatc gtcgctttgt gaaccaacac ctg                                    33


<210>  44
<211>  33
<212>  DNA
<213>  Artificial

<220>
<223>  primer

<400>  44
gacaatgatc gtcgctttgt gaaccaacac ctg                                    33


<210>  45
<211>  33
<212>  DNA
<213>  Artificial

<220>
<223>  primer

<400>  45
ctgaacagcg ctcgctttgt gaaccaacac ctg                                    33


<210>  46
<211>  30
<212>  DNA
<213>  Artificial

<220>
<223>  primer

<400>  46
ggttctcctc gctttgtgaa ccaacacctg                                        30


<210>  47
<211>  21
<212>  DNA
<213>  Artificial

<220>
<223>  primer

<400>  47
ggcattgtgg aacaatgctg t                                                 21


<210>  48
<211>  27
<212>  DNA
<213>  Artificial

<220>
<223>  primer

<400>  48

ctagttgcag tagttctcca gctggta                                          27


<210>  49
<211>  33
<212>  DNA
<213>  Artificial

<220>
<223>  primer

<400>  49
gatggcgacc gtggcattgt ggaacaatgc tgt                                  33


<210>  50
<211>  27
<212>  DNA
<213>  Artificial

<220>
<223>  primer

<400>  50
ttacagttca tctttcacag ctttctg                                          27


<210>  51
<211>  5
<212>  PRT
<213>  Artificial

<220>
<223>  linker


<220>
<221>  misc_feature
<222>  (1)
<223>  Xaa represents Asp, Leu or Gly

<220>
<221>  misc_feature
<222>  (2)
<223>  Xaa represents Gly, Asn, Ser or Leu

<220>
<221>  misc_feature
<222>  (3)
<223>  Xaa represents Asp, Ser or Pro

<220>
<221>  misc_feature
<222>  (4)
<223>  Xaa represents Arg or Ala

<400>  51

Xaa Xaa Xaa Xaa Arg
1               5


<210>  52
<211>  4

<212>  PRT
<213>  Artificial

<220>
<223>  linker


<220>
<221>  misc_feature
<222>  (1)
<223>  Xaa represents Asp, Leu or Gly

<220>
<221>  misc_feature
<222>  (2)
<223>  Xaa represents Gly, Asn, Ser or Leu

<220>
<221>  misc_feature
<222>  (3)
<223>  Xaa represents Asp, Ser or Pro

<400>  52

Xaa Xaa Xaa Arg
1

## Description

### BACKGROUND OF THE INVENTION

### Field of the Invention

The present invention relates to DNA encoding a fusion protein containing highly-expressed and -secreted insulin precursors for producing recombinant insulin, and to a method for producing insulin using such DNA.

### Background Art

Insulin is the only hormone that lowers blood glucose levels in the body. If the insulin-secretory function is lowered for some reason, insulin-dependent diabetes mellitus (IDDM) may be developed. Insulin is a medicine that is indispensable for treatment of patients with lowered insulin-secretory functions.

Human insulin is a polypeptide comprising an A chain, consisting of 21 amino acid residues, and a B chain, consisting of 30 amino acid residues. A single disulfide linkage is present in the A chain, and two disulfide linkages are present between the A chain and the B chain. Insulin is firstly synthesized by the ribosomes in B cells in pancreatic islets of Langerhans as preproinsulin (SP-B-C-A) comprising a signal peptide consisting of 24 amino acid residues (SP), the B chain (B), the C peptide consisting of 31 amino acid residues (C), and the A chain (A) arranged in tandem in the order. When preproinsulin enters the endoplasmic reticulum (ER), the signal peptide is cleaved therefrom to convert preproinsulin into proinsulin (B-C-A). Disulfide linkages are formed in the proinsulin chain in the endoplasmic reticulum (ER), and proinsulin has a three-dimensional structure. Thereafter, a prohormone-converting enzyme, PC1/3, cleaves the B-C linkage, and PC2 cleaves the C-A linkage. Finally, two basic amino acid residues left at the C terminus of the B chain upon the cleavage by PC1/3 are truncated by carboxypeptidase H and a functional insulin is formed.

Although insulins for therapeutic use were initially those extracted from bovine or porcine pancreas; insulins that are currently used for therapeutic purpose are mostly genetically recombinant insulins.

Up to the present, recombinant insulins have been produced by transforming microorganisms with vectors comprising DNAs encoding proinsulin to express proinsulin, allowing formation of disulfide linkages, and converting proinsulin into insulins. Several methods of production involving such procedures have been heretofore developed. Eli Lilly and Company, for example, have developed a method for producing insulin by using *E. coli* to express proinsulin, allowing formation of disulfide linkages *in vitro,* and cleaving the C peptide with trypsin and carboxypeptidase B (JP Patent Publication (kohyo) No. 1-48278 B (1989) and JP Patent No. 2634176).

Novo Nordisk has developed methods for producing insulins by allowing miniproinsulin comprising the B chain connected to the A chain with two basic amino acid residues between them to express in yeast and treating it with trypsin *in vitro* (JP Patent Publication (kohyo) No. 7-121226 B (1995); JP Patent Publication (kohyo) No. 8-8871 B (1996); and JP Patent No. 2553326). These methods are advantageous on the point that disulfide linkage formation *in vitro* is not required due to expression and secretion of disulfide-linked insulin precursors. Additionally, since insulin precursors are secreted in the medium, separation and purification are easier.

Development of novel methods for producing recombinant insulins has been actively attempted. Hoechist (JP Patent Publication (kokai) No. 2-195896 A (1990); JP Patent Publication (kokai) No. 2-225498 A (1990); JP Patent Publication (kokai) No. 2-233698 A (1990); JP Patent Publication (kokai) No. 3-169895 A (1991); JP Patent Publication (kokai) No. 4-258296 A (1992); JP Patent Publication (kokai) No. 6-228191 A (1994); and JP Patent Publication (kokai) No. 7-265092 A (1995)) and BIO-TECHNOLOGY GENERAL CORP. (WO 96/20724) have developed novel production methods using *E. coli.*

Thus, a plurality of approaches exist regarding methods for producing recombinant insulins, and further improvement has been attempted in terms of expression efficiency, efficiency of disulfide linkage formation, and a method for conversion of proinsulins into insulins.

As hosts for producing recombinant proteins, microorganisms are most frequently used from the viewpoint of easy operability and availability for industrial production. In particular, *E. coli* and yeast hosts are well known. The expression systems for recombinant proteins using *Brevibacillus brevis* of the genus *Brevibacillus,* that have been developed recent years, allow expression and secretion of polypeptides which have disulfide linkages in their functional state (e.g. human epidermal growth factors) as active, i.e., disulfide-linked, polypeptides in a medium in large quantity. Thus, such expression systems have drawn attention as systems for large-scale production of recombinant proteins (JP Patent No. 2082727, JP Patent Publication (kokai) No. 62-201583 A (1987); Yamagata, H. et al., J. Bacteriol. 169, 1239-1245, 1987; Shigezo Udaka, Journal of Japan Society for Bioscience, Biotechnology, and Agrochemistry, 61: 669-676, 1987; Takao, M. et al., Appl. Microbiol. Biotechnol. 30, 75-80, 1989; and Yamagata, H. et al., Proc. Natl. Acad. Sci. U.S.A., 86, 3589-3593, 1989).

Expression of insulin precursors was attempted in the gene expression system of *Brevibacillus brevis,* and methods for expression and secretion of proinsulin (JP Patent No. 3313083) and mutant proinsulin (JP Patent No. 3406244) were developed. Thus, the possibility of producing recombinant insulins using *Brevibacillus brevis* as a host was demonstrated.

Furthermore, an attemption for insulin production using such expression system in an industrial scale has been made by means of coexpression with a protein disulfide isomerase (WO 01/068884).

The object of the present invention is to provide an optimal insulin precursor sequence that enables expression and secretion of insulin precursors at higher levels in gene expression systems using bacteria, in particular those of the genus *Bacillus* or the genus *Brevibacillus,* in order to allow production of recombinant insulins in an inductrial scale.

### SUMMARY OF THE INVENTION

The present inventors have conducted concentrated studies and enabled secretion and expression of insulin precursors at high levels in the *Bacillus* or *Brevibacillus* expression systems via, for example, insertion of a sequence consisting of 5, 6, 7, or 12 amino acid residues from the N-terminus of the cell-wall protein (CWP) of a bacterium of the genus *Bacillus* or *Brevibacillus* (i.e., the leader peptide), insertion of a linker peptide between the leader peptide and the insulin B chain, use of a fusion polypeptide of the insulin B chain (lacking Thr at the C terminus) and the A chain, and/or insertion of a linker peptide between the insulin B chain and the A chain, in fusion proteins comprising insulin precursors. This has led to the completion of the present invention. Further, the present inventors confirmed that insulin could be produced in high yields from such precursors.

Specifically, the present invention provides the following.
(1) DNA encoding a fusion protein comprising: a signal peptide from MWP, which is a cell-wall protein (CWP) of a bacterium of the genus *Bacillus* or *Brevibacillus;* a leader peptide comprising 5 to 7 or 12 amino acid residues from CWP of a bacterium of the genus *Bacillus* or *Brevibacillus;* a linker peptide comprising an amino acid sequence represented by the general formula: (Asp, Leu, or Gly)(Gly, Asn, Ser, or Leu)(Asp, Ser, or Pro)(Arg, Ala, or none)Arg (SEQ ID NO: 51 or 52); and an amino acid sequence of an insulin precursor, ligated in the order.
(2) DNA according to (1), wherein the linker peptide comprises an amino acid sequence as shown in any one of SEQ ID NOs: 1 to 6.
(3) DNA according to (1) or (2), wherein the leader peptide is from MWP.
(4) DNA according to any one of (1) to (3), wherein the insulin precursor comprises an amino acid sequence as shown in SEQ ID NO: 8 or 9.
(5) DNA according to any one of (1) to (4), wherein the fusion protein comprises an amino acid sequence as shown in any one of SEQ ID NOs: 10 to 18.
   The amino acid sequences as shown in SEQ ID NOs: 10 to 18 have the structures as follows, respectively:
   MWPsp-MWPmp5-AspGlyAspArgArg-B chain(desThr)-A chain (SEQ ID NO: 10);
   MWPsp-MWPmp6-AspGlyAspArgArg-B chain(desThr)-A chain (SEQ ID NO: 11);
   MWPsp-MWPmp6-LeuAsnSerAlaArg-B chain(desThr)-A chain (SEQ ID NO: 12);
   MWPsp-MWPmp6-GlySerProArg-B chain(desThr)-A chain (SEQ ID NO: 13);
   MWPsp-MWPmp7-AspGlyAspArgArg-B chain(desThr)-A chain (SEQ ID NO: 14);
   MWPsp-MWPmp7-AspLeuAspArgArg-B chain(desThr)-A chain (SEQ ID NO: 15);
   MWPsp-MWPmp7-AspAsnAspArgArg-B chain(desThr)-A chain (SEQ ID NO: 16);
   MWPsp-MWPmpl2-AspGlyAspArgArg-B chain(desThr)-A chain (SEQ ID NO: 17); and
   MWPsp-MWPmp7-AspGlyAspArg-B chain-ArgAspGlyAspArg-A chain (SEQ ID NO: 18),
   wherein MWPsp represents an MWP signal peptide; MWPmp represents an leader peptide from MWP; B chain represents the insulin B chain; B chain(desThr) represents the insulin B chain lacking Thr at the C terminus; and A chain represents the insulin A chain.
(6) DNA according to any one of (1) to (5), which comprises a nucleotide sequence as shown in any one of SEQ ID NOs: 19 to 27.
(7) A vector comprising DNA according to any one of (1) to (6).
(8) The vector according to (7), wherein the DNA is operably linked to a site downstream of a promoter sequence from a bacterium.
(9) The vector according to (8), wherein the promoter is from a bacterium of the genus *Bacillus* or *Brevibacillus.*
(10) The vector according to any one of (7) to (9), which further comprises DNA encoding a protein disulfide isomerase (PDI).
(11) A host cell comprising the vector according to any one of (7) to (10).
(12) The host cell according to (11), which is a bacterium of the genus *Bacillus* or *Brevibacillus.*
(13) The host cell according to (12), wherein the bacterium is *Brevibacillus brevis.*
(14) A method for producing insulin comprising steps of: culturing the host cell according to any one of (11) to (13); allowing expression of a desired fusion protein from the host cell; and recovering the expressed polypeptide from the cell or medium.
(15) The method according to (14), which further comprises a step of enzymatically treating the recovered polypeptide.
(16) The method according to (15), wherein the enzymatic treatment is treatment with trypsin.
(17) The method according to any one of (14) to (16), wherein the polypeptide is recovered from the medium.
(18) A fusion protein having an amino acid sequence as shown in any one of SEQ ID NOs: 10 to 18.

### Definition

The term "a bacterium (bacteria) of the genus *Bacillus* or *Brevibacillus"* used herein refers to any bacterium classified as a bacterium of the genus *Bacillus* or *Brevibacillus,* which is a Gram-positive bacillus. Examples thereof include *Brevibacillus brevis, Bacillus subtilis, Bacillus licheniformis,* and *Bacillus polymyxa,* with *Brevibacillus brevis* being preferable.

The term "MWP" used herein refers to a middle wall protein included in a cell-wall protein (CWP) of bacteria having a three-layer cell wall.

The term "insulin precursor" used herein refers to a polypeptide that can be converted into functional insulin via adequate treatment, such as enzyme treatment, and that comprises at least a B chain or a B chain lacking Thr at the C terminus and an A chain.

The present invention enables, by use of a novel fusion protein, the production of recombinant insulin at 1.5 to 3 times higher levels of expression and secretion than the levels attained by conventional *Bacillus* expression systems using insulin precursors. Such recombinant insulin precursors can be converted into insulin and disulfide linkages, which are necessary for the biological activity of insulin, can be accurately formed upon expression and secretion.

### BRIEF DESCRIPTION OF THE DRAWINGS

Fig. 1 shows the nucleotide sequence of:
   MWPsp-MWPmp5-AspGlyAspArgArg-B chain(desThr)-A chain (SEQ ID NO: 19).
Fig. 2 shows the amino acid sequence of:
   MWPsp-MWPmp5-AspGlyAspArgAg-B chain(desThr)-A chain (SEQ ID NO: 10).
Fig. 3 shows the nucleotide sequence of:
   WPsp-MWPmp6-AspGlyAspArgArg-B chain(desThr)-A chain (SEQ ID NO: 20).
Fig. 4 shows the amino acid sequence of:
   WPsp-MWPmp6-AspGlyAspArgArg-B chain(desThr)-A chain (SEQ ID NO: 11).
Fig. 5 shows the nucleotide sequence of:
   MWPsp-MWPmp6-LeuAsnSerAlaArg-B chain(desThr)-Achain (SEQ ID NO: 21).
Fig. 6 shows the amino acid sequence of:
   MWPsp-MWPmp6-LeuAsnSerAlaArg-B chain(desThr)-A chain (SEQ ID NO: 12).
Fig. 7 shows the nucleotide sequence of:
   MWPsp-MWPmp6-GlySerProArg-B chain(desThr)-A chain (SEQ ID NO: 22).
Fig. 8 shows the amino acid sequence of:
   MWPsp-MWPmp6-GlySerProArg-B chain(desThr)-A chain (SEQ ID NO: 13).
Fig. 9 shows the nucleotide sequence of:
   MWPsp-MWPmp7-AspGlyAspArgArg-B chain(desThr)-A chain (SEQ ID NO: 23).
Fig. 10 shows the amino acid sequence of:
   MWPsp-MWPmp7-AspGlyAspArgArg-B chain(desThr)-A chain (SEQ ID NO: 14).
Fig. 11 shows the nucleotide sequence of:
   MWPsp-MWPmp7-AspLeuAspArgArg-B chain(desThr)-A chain (SEQ ID NO: 24).
Fig. 12 shows the amino acid sequence of:
   MWPsp-MWPmp7-AspLeuAspArgArg-B chain(desThr)-A chain (SEQ ID NO: 15).
Fig. 13 shows the nucleotide sequence of:
   MWPsp-MWPmp7-AspAsnAspArgArg-B chain(desThr)-A chain (SEQ ID NO: 25).
Fig. 14 shows the amino acid sequence of:
   MWPsp-MWPmp7-AspAsnAspArgArg-B chain(desThr)-A chain (SEQ ID NO: 16).
Fig. 15 shows the nucleotide sequence of:
   MWPsp-MWPmp12-AspGlyAspArgArg-B chain(desThr)-A chain (SEQ ID NO: 26).
Fig. 16 shows the amino acid sequence of:
   MWPsp-MWPmp12-AspGlyAspArgArg-B chain(desThr)-A chain (SEQ ID NO: 17).
Fig. 17 shows the nucleotide sequence of:
   MWPsp-MWPmp7-AspGlyAspArg-B chain-ArgAspGlyAspArg-A chain (SEQ ID NO: 27).
Fig. 18 shows the amino acid sequence of:
   MWPsp-MWPmp7-AspGlyAspArg-B chain-ArgAspGlyAspArg-A chain (SEQ ID NO: 18).
Fig. 19 schematically shows a method for incorporating the fusion DNA into the pNU211R2L5 expression vector for *Bacillus brevis.*
Fig. 20 shows the expression levels of fusion proteins.
Fig. 21 shows HPLC elution patterns showing peptide mapping of des-Thr insulin.
Fig. 22 shows HPLC elution patterns of mature insulin.

### Sequence Listing

SEQ ID NOs: 1 to 7: Linkers
SEQ ID NOs: 8 and 9: Insulin precursors
SEQ ID NOs: 10 to 27: Fusion proteins
SEQ ID NOs: 28 to 30: Leader peptides
SEQ ID NOs: 31 to 50: Primers
SEQ ID NOs: 51 and 52: Linkers.

### DETAILED DESCRIPTION OF THE PREFERRED EMBODIMENTS

According to the present invention, expressing DNA encoding the above fusion proteins (hereafter occasionally referred to as "miniproinsulin" or "MINIPINS") in a bacterium of the genus *Bacillus* or *Brevibacillus* allows insulin precursors to be secreted in the medium in a large amount. The resultant is separated and purified and then cleaved with trypsin. If B chain(desThr) is used, Thr may be added to obtain naturally-occurring insulins having desired structures.

The first requirement for expression and secretion of insulin precursors at high levels is the provision of a leader peptide and a linker peptide each comprising a given amino acid sequence between a signal peptide, which is necessary for secretion, and insulin precursors in the fusion protein.

According to an embodiment of the present invention, a preferable leader peptide for high-level expression and secretion of insulin precursors comprises 5, 6, 7, or 12 amino acid residues at the N-terminus of a cell-wall protein (CWP) of a bacterium of the genus *Bacillus* or *Brevibacillus.* Examples of CWP that can be used include, but are not limited to, those from the *Brevibacillus brevis* strains 47 (FERM P-7224: JP Patent Publication (kokai) No. 60-58074 A (1985); JP Patent Publication (kokai) No. 62-201583 A (1987)) and HPD31 (FERM BP-1087: JP Patent Publication (kokai) No. 4-278091 A (1992)). Specific examples of leader peptides include the following sequences (the documents citing the relevant sequences are shown in the parentheses):
MWPmpl2: AlaGluGluAlaAlaThrThrThrAlaProLysMet (SEQ ID NO: 28; Biotechnol., Genet. Eng. Rev., 7: 278-311, 1989);
OWPmp12: AlaProLysAspGlyIleTyrIleGlyGlyAsnIle (SEQ ID NO: 29; J. Bacteriol., 170: 935-945, 1988); and
HWPmp12: AlaGluAspThrThrThrAlaProLysMetAspAla (SEQ ID NO: 30; J. Bacteriol., 172: 1312-1320, 1990).

The number of amino acid residues from the N-terminus is not limited, provided that the fusion proteins of interest are expressed at high levels, and the number of amino acid residues is preferably 5, 6, 7, or 12. Other examples are 8, 10, or 11 amimo acid residues (JP Patent No. 3313083).

Linker peptides that are disposed immediately before the B chain or the B chain lacking Thr at the C terminus (B chain(desThr)) of the insulin precursor and optionally between the B chain and the A chain have two functions. One such function is that as a site cleaved with trypsin for conversion from insulin precursors into insulin. The other function is to express and secrete insulin precursors at high levels. The site to be cleaved with trypsin is a basic amino acid, Arg or Lys. Thus, the C terminus of the linker peptide immediately before the B chain or B chain(desThr) preferably comprises one or two Arg residues. Also, the N terminus and the C terminus of the linker peptide between the B chain and the A chain preferably comprise one or two Arg residues. When the B chain lacking Thr at the C terminus is directly connected to the A chain, Lys, which is the second residue from the C terminus of the B chain, serves as the cleavage site.

A linker peptide comprising one or more amino acid residues is generally present between functional domains in a protein, and such linker peptide connects domains without influencing the functions of such domains. In the present invention, a linker peptide is disposed between a leader peptide and the B chain or B chain(desThr) and optionally between the B chain and the A chain via trypsin cleavage sites. Also, a linker peptide is useful for facilitating formation of disulfide linkages between the B chain and the A chain and/or within the A chain, cleavage with trypsin, and expression of the fusion protein of interest. As long as such functions are maintained, a linker peptide may comprise one or more amino acids, and a linker peptide is not required to have a specific amino acid sequence. Preferably, however, such linker peptide is: (i) a linker peptide disposed between the leader peptide and the B chain and having the amino acid sequence represented by the following general formula: (Asp, Leu, or Gly)(Gly, Asn, Ser, or Leu)(Asp, Ser, or Pro)(Arg, Ala, or none)Arg (SEQ ID NO: 51 or 52), and more preferably a sequence AspGlyAspArgArg (SEQ ID NO: 1), LeuAsnSerAlaArg (SEQ ID NO: 2), GlySerProArg (SEQ ID NO: 3), AspLeuAspArgArg (SEQ ID NO: 4), AspAsnAspArgArg (SEQ ID NO: 5), or AspGlyAspArg (SEQ ID NO: 6); and (ii) a linker peptide disposed between the B chain and the A chain having a sequence represented by ArgAspGlyAspArg (SEQ ID NO: 7). Preferably, a linker peptide between the B chain and the A chain may be absent when the B chain(desThr) is used.

DNA of the present invention can be prepared by employing techniques known in the art in combination. For example, DNA of interest can be generated by preparing each DNA sequence of the components independently by means of chemical synthesis or cloning, connecting the components sequentially with the use of a ligase, and performing PCR amplification techniques. More specifically, the details of such techniques would be understood with reference to the Examples below. Examples of general techniques that can be performed include those described in Maniatis, T. et al., Molecular Cloning, A Laboratory Manual, 2nd ed., Cold Spring Harbor Laboratory, 1989, and in Innis, M. A. et al., PCR Protocols, A guide to methods and applications, Academic Press, 1990.

DNA that encodes human proinsulin comprising the insulin B chain, C peptide, and A chain can be obtained from commercially available human pancreatic mRNA with the use of, for example, a commercially available First Strand cDNA Synthesis Kit. If short-strand DNA as a primer can be synthesized using a commercially available DNA synthesizer based on a known DNA sequence (GenBank Accession No. NM_000207), a DNA fragment of interest that encodes the B chain, the A chain, or the like can be amplified via a common polymerase chain reaction (PCR). In such a case, preferably, a cycle of DNA denaturation (94°C for 30 seconds to 2 minutes), annealing to a primer (55°C for 30 seconds to 1 minute), and extension (72°C for 30 seconds to 1 minute) is repeated at least 20 times.

The present invention further provides a vector comprising the DNA of the present invention. A vector that can be used is required at least to have adequate insertion sites into which the DNA of the present invention can be incorporated (i.e., restriction enzyme sites), to be able to express the DNA in a host cell, and to be able to autonomously replicate in the host cell. A vector can comprise a promoter, a replication origin and a terminator sequence, and a vector may further comprise a selection marker such as a drug-resistant gene and a gene that complements auxotrophy. According to an embodiment of the present invention, DNA encoding a fusion protein of interest is operably linked to the 3' terminus of a DNA sequence comprising a promoter region from the genus *Bacillus* or *Brevibacillus.* In the present invention, the term "operably linked" refers to a condition in which a functional nucleotide sequence (e.g., a promoter) is connected in such a manner that the function thereof can be exhibited. Examples of promoters that can be used include glycolytic promoters, tissue-specific promoters, virus promoters, and inducible promoters. Preferable examples of promoters include, but are not limited to, MWP promoters from *Brevibacillus brevis* strain 47 (JP Patent Publication (kohyo) Nos. 1-58950 B (1989) and 7-108224 B (1995)) and HWP promoters from *Brevibacillus brevis* strain HPD31 (JP Patent Publication (kokai) Nos. 4-278091 A (1992) and 6-133782 A (1994)). Examples of selection markers include drug-resistant genes, such as ampicillin-resistant genes, kanamycin-resistant genes, erythromycin-resistant genes, and tetracycline-resistant genes.

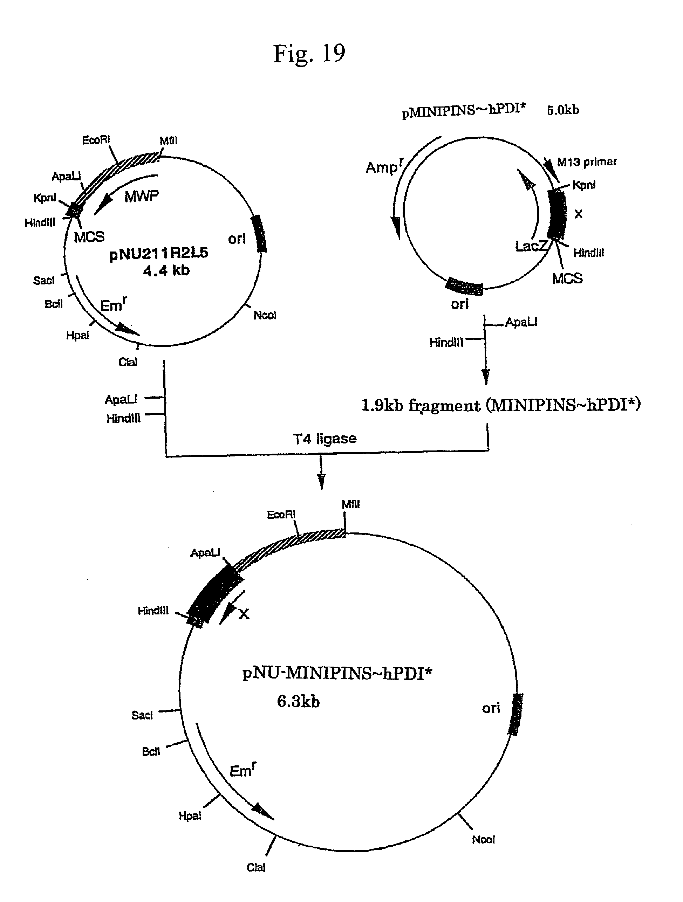

The vector of the present invention may be a DNA vector that is maintained in a compatible prokaryotic or eukaryotic host cell, such as a bacterial, fungal, yeast, insect, plant, or animal cell, and from which a protein of interest can be expressed in these cells. Preferably, the vector of the present invention is a plasmid that can be replicated in a bacterium of the genus *Bacillus* or *Brevibacillus.* Examples of plasmid vectors that can be used include, but are not limited to, pNU200 and pHY500 (Proc. Natl. Acad. Sci. USA, 86: 3589-3593, 1989), pHY4831 (J. Bacteriol., 169: 1239-1245, 1987), pNU100 (Appl. Microbiol. Biotechnol., 30: 75-80, 1989), pNU211 (J. Biochem., 112: 488-491, 1992), pNU211R2L5 (JP Patent Publication (kokai) No. 7-170984 A (1995)), pHY700 (JP Patent Publication (kokai) No. 4-278091 A (1992)), pHT210 (JP Patent Publication (kokai) No. 6-133782 A (1994)), and pHT110R2L5 (Appl. Microbiol. Biotechnol., 42: 358-363, 1994). According to a specific example of the present invention, pNU-MINIPINS-hPDI* expression vectors can be prepared by the method shown in Fig. 19.

The present invention provides a vector that further comprises DNA encoding a protein disulfide isomerase (PDI), in addition to the above DNA. The pNU-MINIPINS∼hPDI* expression vector mentioned above as a specific example carries a PDI gene and thus is useful for constructing a vector comprising DNA encoding a PDI gene. Such vector allows coexpression of a PDI gene and a fusion protein of interest.

Coexpression of PDI with a desired protein is advantageous in the following respect. When expression of a functional polypeptide from a mammal in a bacterial expression system is intended, for example, disulfide linkages required for a functional folding of the polypeptide may not be accurately formed in many cases. Coexpression of PDI upon expression of a polypeptide that would not accurately form or would have difficulty forming disulfide linkages in the bacterial expression system enables construction of an environment in which a polypeptide of interest and PDI are both present and thereby enhances production efficiency of polypeptides having accurate disulfide linkages and desired functions.

Such attempt has been described in WO 01/068884 in detail.

In the present invention, any DNA from mammals, including humans, and DNA from eukaryotic organisms, such as insects or yeast can be employed as DNA encoding PDI. The nucleotide sequences of mammalian PDI genes, such as those of humans (N_000918), mice (NM_011032), and rats (NM_012998), are registered in the database. The yeast PDI is described in, for example, WO 98/035049.

Preferable hosts for the use of the above-described PDI coexpression system are bacteria of the genus *Bacillus* or *Brevibacillus,* with *Brevibacillus brevis* being the most preferable.

The present invention further provides a host cell, such as a bacterial, fungal, yeast, insect, plant, or animal cell, transformed with the vector defined above. Preferably, such host cell is bacteria of the genus *Bacillus* or *Brevibacillus.* Examples of bacteria of the genus *Bacillus* or *Brevibacillus* that can be used as host cells include, but are not limited to, the *Brevibacillus brevis* strains 47 (FERM P-7224: JP Patent Publication (kokai) No. 60-58074 A (1985); JP Patent Publication (kokai) No. 62-201583 A (1987)), 47K (JP Patent Publication (kokai) No. 2-257876 A (1990)), 31-OK (JP Patent Publication (kokai) No. 6-296485 A (1994)), and HPD31 (FERM BP-1087; JP Patent Publication (kokai) No. 4-278091 A (1992)).

The expression vector obtained in the above-described manner may be introduced into a competent host cell, the cell may be cultured in an adequate medium under conditions that allow expression, and recombinant polypeptides of interest may be produced extracellularly or intracellularly, preferably extracellularly. Subsequently, polypeptides may be recovered and purified with a conventional technique.

Examples of methods for vector introduction include calcium phosphate method, electroporation (Methods in Enzymol., 217: 23-33, 1993), spheroplast fusion, protoplast fusion, microinjection, agrobacterium method, and particle gun method.

When culturing the cells containing the expression vector, a person skilled in the art can adequately select known medium and culture conditions in accordance with cell type. When a host cell is a bacterium of the genus *Bacillus* or *Brevibacillus,* for example, culture of the host cell may be conducted in T2 medium at 37°C for 1 day, aliquot of the cell suspension in T2 medium may be transferred to M-5YC medium, and the medium may then be subjected to shaking culture at 30°C for 4 days.

Polypeptides produced extracellularly can be recovered from, for example, a medium in which the cells were cultured. Polypeptides produced intracellularly can be recovered, for example, by collecting cells via centrifugation, disrupting the cells, and then recovering the polypeptides.

The recovered polypeptides can be purified via, for example, gel filtration chromatography, ion-exchange chromatography, affinity chromatography, hydrophobic interaction chromatography, electrophoresis, or isoelectric focusing, and such techniques may be carried out alone or in combinations of two or more.

The polypeptides obtained as above are then subjected to trypsin treatment to prepare desThr-insulin, and they are further subjected to trypsin treatment in the presence of tert-butyl-Thr to obtain insulin. Thus, the present invention further provides a method for producing insulin comprising culturing bacteria of the genus *Bacillus* or *Brevibacillus* transformed as described above in a medium, allowing accumulation of polypeptides comprising insulin sequences outside the cells, and subjecting the recovered polypeptides to trypsin treatment to obtain insulin.

The recombinant insulin thus obtained has disulfide linkages and an HPLC elution pattern that are identical to those of naturally-occurring insulin. Thus, such recombinant insulin is useful for a therapeutic drug for insulin-dependent diabetes mellitus.

### Examples

Hereafter, the present invention is described in detail with reference to the following examples, although the technical scope of the present invention is not limited thereto.

DNA encoding a fusion protein was prepared by ligating DNA fragments amplified via polymerase chain reaction (PCR) by ligation with the use of DNA ligase. In the description, "MWPsp" refers to a signal peptide of an MWP protein, "MWPmp" refers to an N-terminal peptide of an MWP mature protein, and a following numerical value refers to the number of amino acid residues from the N terminus.

### Example I

### 1. Preparation of various DNA fragments

### (1) Preparation of DNA fragment of MWPsp-MWPmp5

### a. Template DNA

Genomic DNA (840 ng) extracted from *Brevibacillus brevis* (47-5Q strain) in accordance with a conventional technique (Molecular Cloning, A Laboratory Manual, 2nd ed., Cold Spring Harbor Laboratory, 1989) was used.

### b. Primers

Forward primer: 5'-ACACGCGCTTGCAGGATTCG-3' (SEQ ID NO: 31)
Reverse primer: 5'-TGCTGCTTCTTCTGCTGC-3' (SEQ ID NO: 32)

Primers were obtained based on the nucleotide sequences of the MWP protein determined by Yamagata, H. et al. (J. Bacteriol., 169, 1239-1245, 1987) and Tsuboi, A. et al. (J. Bacteriol., 170, 935-945, 1988) by organic chemical synthesis and added to a final concentration of 0.1 µmol/l in the reaction solution.

### c. Taq DNA polymerase

Five units of a commercially available Taq DNA polymerase (GIBCO BRL) was added per reaction.

### d. Others

Tris-HCl (final concentration: 20 mmol/l, pH 8), MgCl₂ (final concentration: 2.5 mmol/l), and dNTPs (dATP, dGTP, dCTP and dTTP; final concentration: 50 µmol/l each) were added.

Components (a) to (d) above were introduced into a 0.5-ml tube so as to bring the amount of the reaction solution to 100 µl, and PCR was carried out (30 cycles of denaturation at 94°C for 1 minute, annealing at 50°C for 1 minute, and extension of DNA chain at 72°C for 1 minute) in accordance with a conventional technique (Innis, M. A. et al., PCR Protocols, A guide to methods and applications, Academic Press, 1990). After the completion of PCR, the reaction solution was concentrated with phenol, applied on 0.8% agarose gel, and electrophoresed under conditions commonly used. The PCR product, i.e., the DNA fragment of MWPsp-MWPmp5, was recovered from agarose gel using Millipore Ultrafree-C3H. The recovered PCR product was subjected to phenol extraction, ethanol precipitation, and vacuum drying. The product was then dissolved in an adequate amount of distilled water. The DNA blunting kit (Takara Shuzo Co., Ltd.) was used to perform blunt-ending in accordance with the manufacturer's instructions.

### (2) Preparation of DNA fragment of MWPsp-MWPmp6

A blunt-ended DNA fragment of MWPsp-MWPmp6 was obtained in accordance with the procedure as described in (1) above, except for the following respects.

The reverse primer 5'-AGTTGCTGCTTCTTCTGC-3' (SEQ ID NO: 33) was used.

A PCR cycle of denaturation at 94°C for 1 minute, annealing at 50°C for 1 minute, and extension of the DNA strand at 72°C for 30 seconds was repeated 25 times.

### (3) Preparation of DNA fragment of MWPsp-MWPmp7

A blunt-ended DNA fragment of MWPsp-MWPmp7 was obtained in accordance with the procedure as described in (1) above, except for the following respects.

The reverse primer 5'-AGTAGTTGCTGCTTCTTC-3' (SEQ ID NO: 34) was used.

A PCR cycle of denaturation at 94°C for 1 minute, annealing at 50°C for 1 minute, and extension of the DNA strand at 72°C for 30 seconds was repeated 25 times.

### (4) Preparation of DNA fragment of MWPsp-MWPmp12

A blunt-ended DNA fragment of MWPsp-MWPmp12 was obtained in accordance with the procedure as described in (1) above, except for the following respects.

The reverse primer 5'-CATTTTTGGAGCTGTAGT-3' (SEQ ID NO: 35) was used.

A PCR cycle of denaturation at 94°C for 1 minute, annealing at 50°C for 1 minute, and extension of the DNA strand at 72°C for 30 seconds was repeated 25 times.

### (5) Preparation of DNA fragment of proinsulin

A blunt-ended DNA fragment of proinsulin was obtained in accordance with the procedure as described in (1) above, except for the following respects.

As template DNA, 10 ng of a plasmid vector comprising human preproinsulin DNA incorporated therein was used. A plasmid vector comprising human preproinsulin DNA incorporated therein was obtained in the following manner. From commercialized human pancreatic mRNA (Clontech), human pancreatic cDNA was synthesized using the First Strand cDNA Synthesis Kit (Pharmacia) in accordance with the manufacturer's instructions. With the use of the resulting cDNA as a template, PCR was carried out using the forward primer 5'-ATGGCCCTGTGGATGCGCC-3' (SEQ ID NO: 36) and the reverse primer 5'-CTAGTTGCAGTAGTTCTCC-3' (SEQ ID NO: 37), which were synthesized based on the nucleotide sequences of the human preproinsulin gene determined by Bell, G. I. et al. (Nature, 282, 525-527, 1979) (a cycle of 94°C for 1 minute, 60°C for 1 minute, and 72°C for 1 minute was repeated 35 times). The resulting PCR product, i.e., human preproinsulin DNA, was cloned into the pGEM-T vector (Promega).

The forward primer 5'-TTTGTGAACCAACACCTG-3' (SEQ ID NO: 38) and the reverse primer 5'-CTAGTTGCAGTAGTTCTCC-3' (SEQ ID NO: 37) were used.

A PCR cycle of denaturation at 94°C for 1 minute, annealing at 47°C for 1 minute, and extension of the DNA strand at 72°C for 30 seconds was repeated 25 times.

### (6) Preparation of DNA fragment of DGDR-B chain-R

A blunt-ended DNA fragment of DGDR-B chain-R was obtained in accordance with the procedure as described in (1) above, except for the following respects.

As template DNA, 10 ng of the proinsulin PCR product obtained in (5) above was used.

The forward primer 5'-GACGGTGATCGCTTTGTGAACCAACACCTG-3' (SEQ ID NO: 39) and the reverse primer 5'-GCGGGTCTTGGGTGTGTAGAA-3' (SEQ ID NO: 40) were used.

A PCR cycle of denaturation at 94°C for 1 minute, annealing at 52°C for 1 minute, and extension of the DNA strand at 72°C for 30 seconds was repeated 25 times.

### (7) Preparation of DNA fragment of DGDRR-B chain(desThr)

A blunt-ended DNA fragment of DGDRR-B chain(desThr) was obtained in accordance with the procedure as described in (1) above, except for the following respects. Further, a phosphorylated DNA fragment of DGDRR-B chain(desThr) was obtained by phosphorylation using T4 polynucleotide kinase (Nippon Gene) in accordance with the manufacturer's instructions.

As template DNA, 10 ng of the proinsulin PCR product obtained in (5) above was used.

The forward primer 5'-GACGGTGATCGTCGCTTTGTGAACCAACAC-3' (SEQ ID NO: 41) and the reverse primer 5'-CTTGGGTGTGTAGAAGAA-3' (SEQ ID NO: 42) were used.

A PCR cycle of denaturation at 94°C for 1 minute, annealing at 52°C for 1 minute, and extension of the DNA strand at 72°C for 30 seconds was repeated 25 times.

### (8) Preparation of DNA fragment of DLDRR-B chain(desThr)

A blunt-ended DNA fragment of DLDRR-B chain(desThr) was obtained in accordance with the procedure as described in (1) above, except for the following respects.

As template DNA, 10 ng of the proinsulin PCR product obtained in (5) above was used.

The forward primer 5'-GACTTGGATCGTCGCTTTGTGAACCAACACCTG-3' (SEQ ID NO: 43) and the reverse primer 5'-CTTGGGTGTGTAGAAGAA-3' (SEQ ID NO: 42) were used.

A PCR cycle of denaturation at 94°C for 1 minute, annealing at 52°C for 1 minute, and extension of the DNA strand at 72°C for 30 seconds was repeated 25 times.

### (9) Preparation of DNA fragment of DNDRR-B chain(desThr)

A blunt-ended DNA fragment of DNDRR-B chain(desThr) was obtained in accordance with the procedure as described in (1) above, except for the following respects.

As template DNA, 10 ng of the proinsulin PCR product obtained in (5) above was used.

The forward primer 5'-GACAATGATCGTCGCTTTGTGAACCAACACCTG-3' (SEQ ID NO: 44) and the reverse primer 5'-CTTGGGTGTGTAGAAGAA-3' (SEQ ID NO: 42) were used.

A PCR cycle of denaturation at 94°C for 1 minute, annealing at 52°C for 1 minute, and extension of the DNA strand at 72°C for 30 seconds was repeated 25 times.

### (10) Preparation of DNA fragment of LNSAR-B chain(desThr)

A blunt-ended DNA fragment of LNSAR-B chain(desThr) was obtained in accordance with the procedure as described in (1) above, except for the following respects.

As template DNA, 10 ng of the proinsulin PCR product obtained in (5) above was used.

The forward primer 5'-CTGAACAGCGCTCGCTTTGTGAACCAACACCTG-3' (SEQ ID NO: 45) and the reverse primer 5'-CTTGGGTGTGTAGAAGAA-3' (SEQ ID NO: 42) were used.

A PCR cycle of denaturation at 94°C for 1 minute, annealing at 52°C for 1 minute, and extension of the DNA strand at 72°C for 30 seconds was repeated 25 times.

### (11) Preparation of DNA fragment of GSPR-B chain(desThr)

A blunt-ended DNA fragment of GSPR-B chain(desThr) was obtained in accordance with the procedure as described in (1) above, except for the following respects.

As template DNA, 10 ng of the proinsulin PCR product obtained in (5) above was used.

The forward primer 5'-GGTTCTCCTCGCTTTGTGAACCAACACCTG-3' (SEQ ID NO: 46) and the reverse primer 5'-CTTGGGTGTGTAGAAGAA-3 (SEQ ID NO: 42) were used.

A PCR cycle of denaturation at 94°C for 1 minute, annealing at 52°C for 1 minute, and extension of the DNA strand at 72°C for 30 seconds was repeated 25 times.

### (12) Preparation of DNA fragment of A chain

A blunt-ended DNA fragment of A chain was obtained in accordance with the procedure as described in (1) above, except for the following respects. Further, a phosphorylated DNA fragment of the A chain was obtained by phosphorylation using T4 polynucleotide kinase (Nippon Gene) in accordance with the manufacturer's instructions.

As template DNA, 10 ng of the proinsulin PCR product obtained in (5) above was used.

The forward primer 5'-GGCATTGTGGAACAATGCTGT-3' (SEQ ID NO: 47) and the reverse primer 5'-CTAGTTGCAGTAGTTCTCCAGCTGGTA-3' (SEQ ID NO: 48) were used.

A PCR cycle of denaturation at 94°C for 1 minute, annealing at 55°C for 1 minute, and extension of the DNA strand at 72°C for 30 seconds was repeated 25 times.

### (13) Preparation of DNA fragment of DGDR-A chain

A blunt-ended DNA fragment of DGDR-A chain was obtained in accordance with the procedure as described in (1) above, except for the following respects. Further, a phosphorylated DNA fragment of the DGDR-A chain was obtained by phosphorylation using T4 polynucleotide kinase (Nippon Gene) in accordance with the manufacturer's instructions.

As template DNA, 10 ng of the DNA fragment of A chain obtained in (12) above was used.

The forward primer 5'-GATGGCGACCGTGGCATTGTGGAACAATGCTGT-3' (SEQ ID NO: 49) was used.

A PCR cycle of denaturation at 94°C for 1 minute, annealing at 55°C for 1 minute, and extension of the DNA strand at 72°C for 30 seconds was repeated 25 times.

### 2. Preparation of the fusion DNAs of MWPsp-MWPmp5, 6, 7, or 12 with various DNA fragments of B chain

### (1) Preparation of fusion DNA of MWPsp-MWPmp7-DGDR-B chain-R

A blunt-ended fusion DNA of MWPsp-MWPmp7-DGDR-B chain-R was prepared in accordance with the procedure as described in 1 (1), except for the following respects.

Adequate amounts of the DNA fragment of MWPsp-MWPmp7 obtained in 1 (3) and the DNA fragment of DGDR-B chain-R obtained in 1 (6) were mixed, the reaction was carried out at 16°C for 30 minutes using the DNA Ligation Kit (Takara Shuzo Co., Ltd.), and the resultant was used as template DNA.

The reverse primer 5'-GCGGGTCTTGGGTGTGTAGAA-3' (SEQ ID NO: 40) was used.

Subsequently, the blunt-ended PCR product was subjected to phosphorylation using T4 polynucleotide kinase (Nippon Gene) in accordance with the manufacturer's instructions. The product was incorporated into the HincII-cleaved BlueScript plasmid vector (Stratagene), the DNA nucleotide sequence was determined, and formation of desired fusion DNA was confirmed.

### (2) Preparation of fusion DNA of MWPsp-MWPmp5-DGDRR-B chain(desThr)

A blunt-ended fusion DNA of MWPsp-MWPmp5-DGDRR-B chain(desThr) was obtained in accordance with the procedure as described in 1 (1), except for the following respects.

Adequate amounts of the DNA fragment of MWPsp-MWPmp5 obtained in 1 (1) and the DNA fragment of DGDRR-B chain(desThr) obtained in 1 (7) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (3) Preparation of fusion DNA of MWPsp-MWPmp6-DGDRR-B chain(desThr)

A blunt-ended fusion DNA of MWPsp-MWPmp6-DGDRR-B chain(desThr) was obtained in accordance with the procedure as described in 1 (1), except for the following respects.

Adequate amounts of the DNA fragment of MWPsp-MWPmp6 obtained in 1 (2) and the DNA fragment of DGDRR-B chain(desThr) obtained in 1 (7) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (4) Preparation of fusion DNA of WPsp-MWPmp7-DGDRR-B chain(desThr)

A blunt-ended fusion DNA of MWPsp-MWPmp7-DGDRR-B chain(desThr) was obtained in accordance with the procedure as described in 1 (1), except for the following respects.

Adequate amounts of the DNA fragment of MWPsp-MWPmp7 obtained in 1 (3) and the DNA fragment DGDRR-B chain(desThr) obtained in 1 (7) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (5) Preparation of fusion DNA of MWPsp-MWPmp12-DGDRR-B chain(desThr)

A blunt-ended fusion DNA of MWPsp-MWPmp12-DGDRR-B chain(desThr) was obtained in accordance with the procedure as described in 1 (1), except for the following respects.

Adequate amounts of the DNA fragment of MWPsp-MWPmp12 obtained in 1 (4) and the DNA fragment DGDRR-B chain(desThr) obtained in 1 (7) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (6) Preparation of fusion DNA of MWPsp-MWPmp6-LNSAR-B chain(desThr)

A blunt-ended fusion DNA of MWPsp-MWPmp6-LNSAR-B chain(desThr) was obtained in accordance with the procedure as described in 1 (1), except for the following respects.

Adequate amounts of the DNA fragment of MWPsp-MWPmp6 obtained in 1 (2) and the DNA fragment of LNSAR-B chain(desThr) obtained in 1 (10) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (7) Preparation of fusion DNA of MWPsp-MWPmp6-GSPR-B chain(desThr)

A blunt-ended fusion DNA of MWPsp-MWPmp6-GSPR-B chain(desThr) was obtained in accordance with the procedure as described in 1 (1), except for the following respects.

Adequate amounts of the DNA fragment of MWPsp-MWPmp6 obtained in 1 (2) and the DNA fragment of GSPR-B chain(desThr) obtained in 1 (11) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (8) Preparation of fusion DNA of MWPsp-MWPmp7-DLDRR-B chain(desThr)

A blunt-ended fusion DNA of MWPsp-MWPmp7-DLDRR-B chain(desThr) was obtained in accordance with the procedure as described in 1 (1), except for the following respects.

Adequate amounts of the DNA fragment of MWPsp-MWPmp7 obtained in 1 (3) and the DNA fragment of DLDRR-B chain(desThr) obtained in 1 (8) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### (9) Preparation of fusion DNA of MWPsp.-MWPmp7-DNDRR-B chain(desThr)

A blunt-ended fusion DNA of MWPsp-MWPmp7-DNDRR-B chain(desThr) was obtained in accordance with the procedure as described in 1 (1), except for the following respects.

Adequate amounts of the DNA fragment MWPsp-MWPmp7 obtained in 1 (3) and the DNA fragment DNDRR-B chain(desThr) obtained in 1 (9) were mixed, the reaction was carried out at 16°C for 30 minutes using the DNA Ligation Kit (Takara Shuzo Co., Ltd.), and the resultant was used as template DNA.

The reverse primer 5'-CTTGGGTGTGTAGAAGAA-3' (SEQ ID NO: 42) was used.

In the same manner as in 2 (1), formation of desired fusion DNA was confirmed.

### 3. Preparation of fusion DNA of MWPsp-MWPmp5, 6, 7, or 12 with various DNA fragments of B chain and various DNA fragments of A chain (MINIPINS)

### (1) Preparation of fusion DNA of MWPsp-MWPmp7-DGDR-B chain-RDGDR-A chain

A blunt-ended fusion DNA of MWPsp-MWPmp7-DGDR-B chain-RDGDR-A chain was obtained in accordance with the procedure as described in 1 (1), except for the following respects.

Adequate amounts of the DNA fragment of MWPsp-MWPmp7-DGDR-B chain-R obtained in 2 (1) and the DNA fragment of DGDR-A chain obtained in 1 (13) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (2) Preparation of fusion DNA of MWPsp-MWPmp5-DGDRR-B chain(desThr)-A chain

A blunt-ended fusion DNA of MWPsp-MWPmp5-DGDRR-B chain(desThr)-A chain was obtained in accordance with the procedure as described in 1 (1), except for the following respects.

Adequate amounts of the DNA fragment of MWPsp-MWPmp5-DGDRR-B chain(desThr) obtained in 2 (2) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (3) Preparation of fusion DNA of MWPsp-MWPmp6-DGDRR-B chain(desThr)-A chain

A blunt-ended fusion DNA of MWPsp-MWPmp6-DGDRR-B chain(desThr)-A chain was obtained in accordance with the procedure as described in 1 (1), except for the following respects.

Adequate amounts of the DNA fragment of MWPsp-MWPmp6-DGDRR-B chain(desThr) obtained in 2 (3) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (4) Preparation of fusion DNA of MWPsp-MWPmp7-DGDRR-B chain(desThr)-A chain

A blunt-ended fusion DNA of MWPsp-MWPmp7-DGDRR-B chain(desThr)-A chain was obtained in accordance with the procedure as described in 1 (1), except for the following respects.

Adequate amounts of the DNA fragment of MWPsp-MWPmp7-DGDRR-B chain(desThr) obtained in 2 (4) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (5) Preparation of fusion DNA of MWPsp-MWPmp12-DGDRR-B chain(desThr)-A chain

A blunt-ended fusion DNA of MWPsp-MWPmp12-DGDRR-B chain(desThr)-A chain was obtained in accordance with the procedure as described in 1 (1), except for the following respects.

Adequate amounts of the DNA fragment of MWPsp-MWPmp12-DGDRR-B chain(desThr) obtained in 2 (5) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (6) Preparation of fusion DNA of MWPsp-MWPmp6-LNSAR-B chain(desThr)-A chain

A blunt-ended fusion DNA of MWPsp-MWPmp6-LNSAR-B chain(desThr)-A chain was obtained in accordance with the procedure as described in 1 (1), except for the following respects.

Adequate amounts of the DNA fragment of MWPsp-MWPmp6-LNSAR-B chain(desThr) obtained in 2 (6) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (7) Preparation of fusion DNA of MWPsp-MWPmp6-GSPR-B chain(desThr)-A chain

A blunt-ended fusion DNA of MWPsp-MWPmp6-GSPR-B chain(desThr)-A chain was obtained in accordance with the procedure as described in 1 (1), except for the following respects.

Adequate amounts of the DNA fragment of MWPsp-MWPmp6-GSPR-B chain(desThr) obtained in 2 (7) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (8) Preparation of fusion DNA of MWPsp-MWPmp7-DLDRR-B chain(desThr)-A chain

A blunt-ended fusion DNA of MWPsp-MWPmp7-DLDRR-B chain(desThr)-A chain was obtained in accordance with the procedure as described in 1 (1), except for the following respects.

Adequate amounts of the DNA fragment of MWPsp-MWPmp7-DLDRR-B chain(desThr) obtained in 2 (8) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

In the same manner as in 2 (1), formation of desired fusion DNA was confirmed.

### (9) Preparation of fusion DNA of MWPsp-MWPmp7-DNDRR-B chain(desThr)-A chain

A blunt-ended fusion DNA of MWPsp-MWPmp7-DNDRR-B chain(desThr)-A chain was obtained in accordance with the procedure as described in 1 (1), except for the following respects.

Adequate amounts of the DNA fragment of MWPsp-MWPmp7-DNDRR-B chain(desThr) obtained in 2 (9) and the DNA fragment of A chain obtained in 1 (12) were mixed, the reaction was carried out at 16°C for 30 minutes using the DNA Ligation Kit (Takara Shuzo Co., Ltd.), and the resultant was used as template DNA.

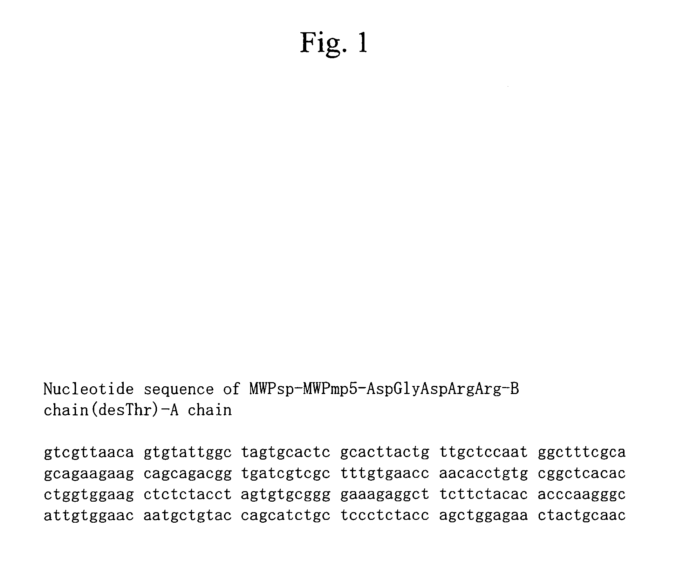

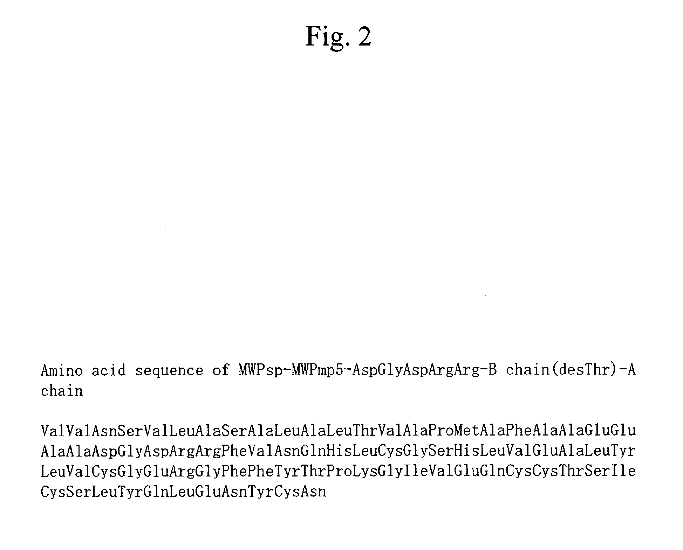

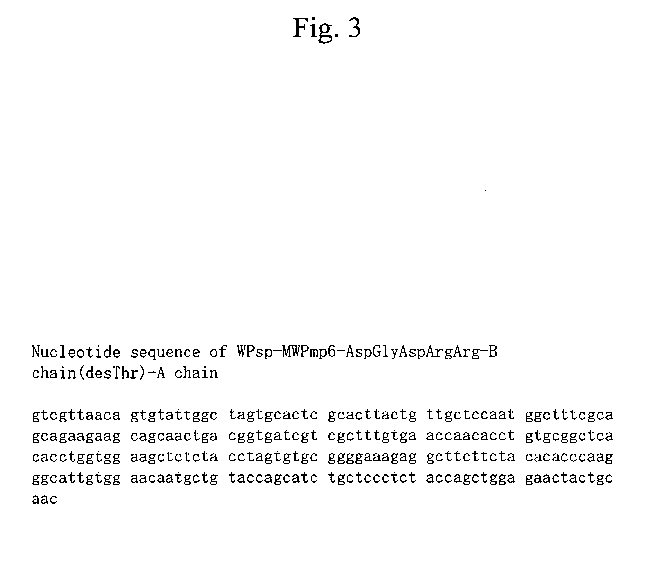

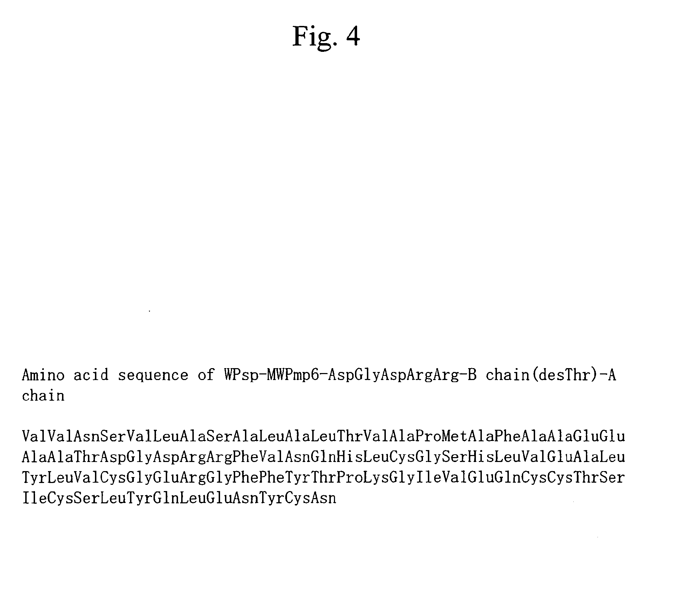

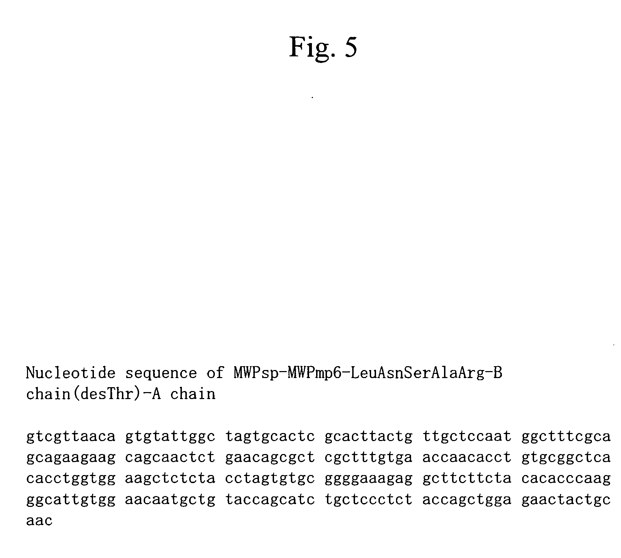

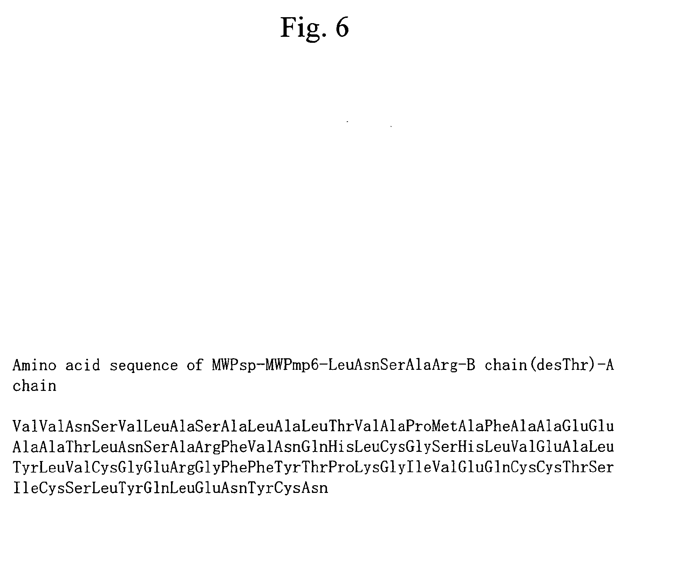

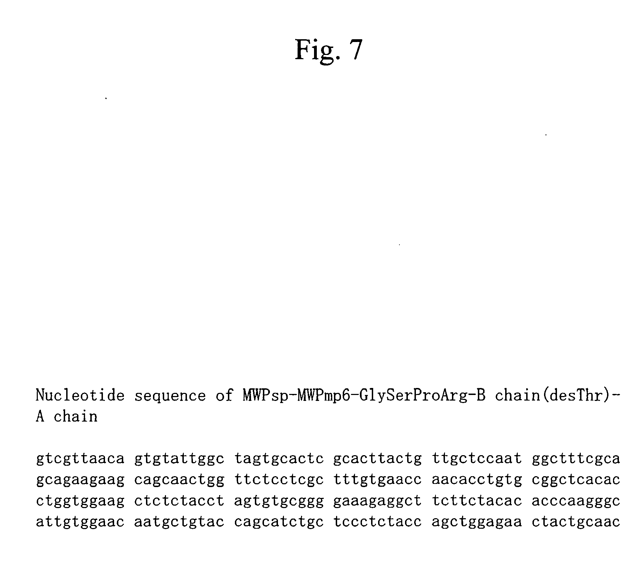

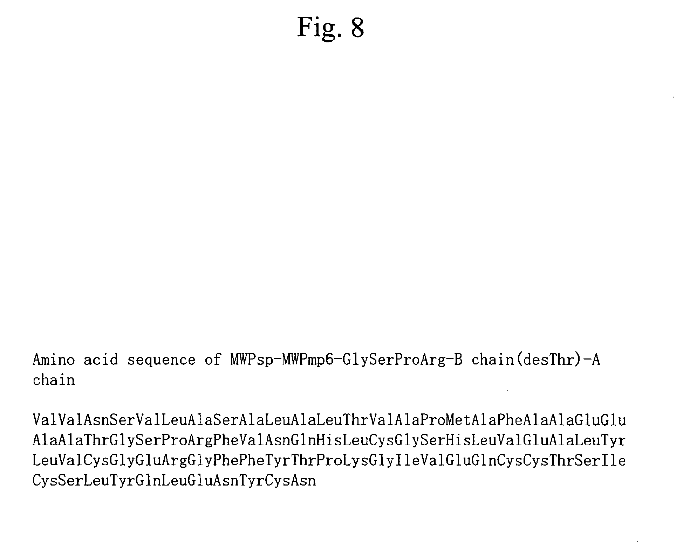

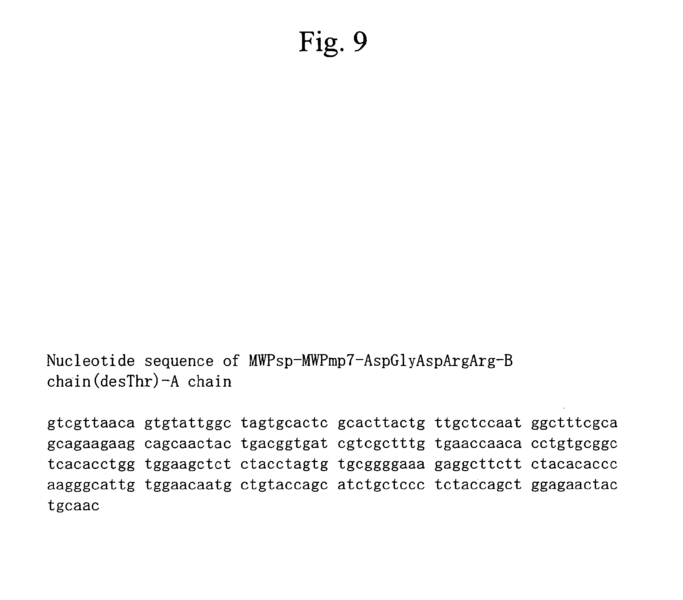

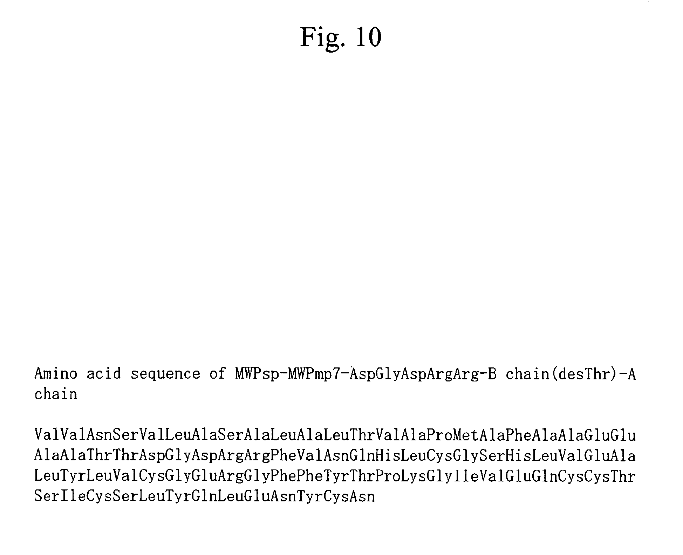

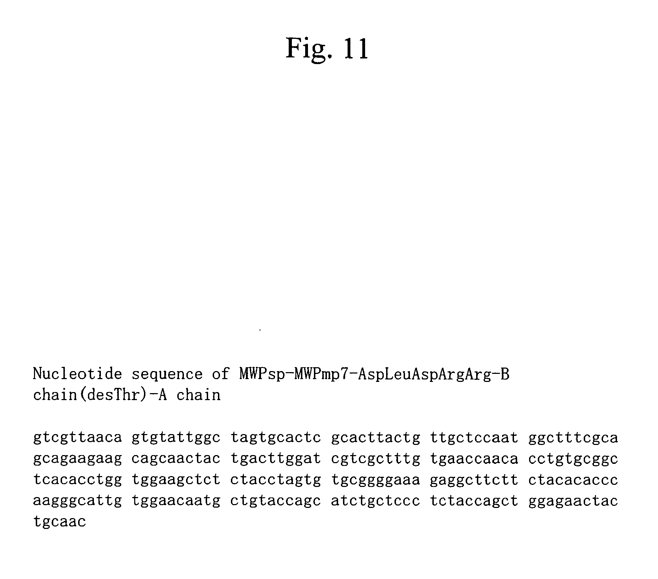

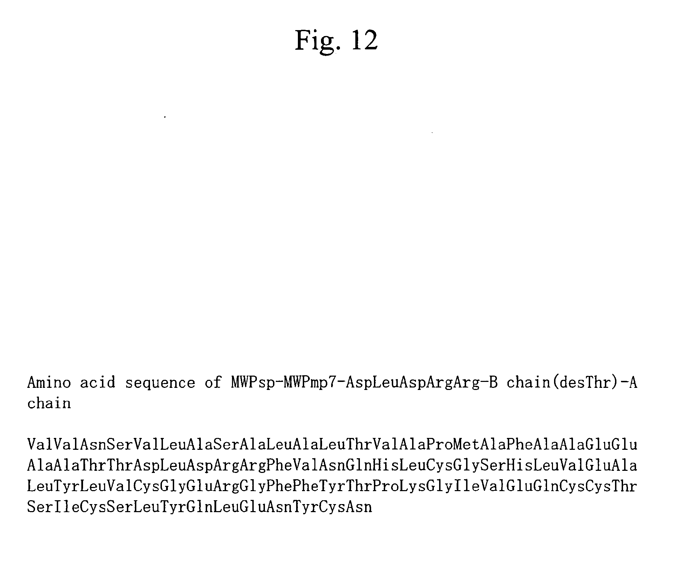

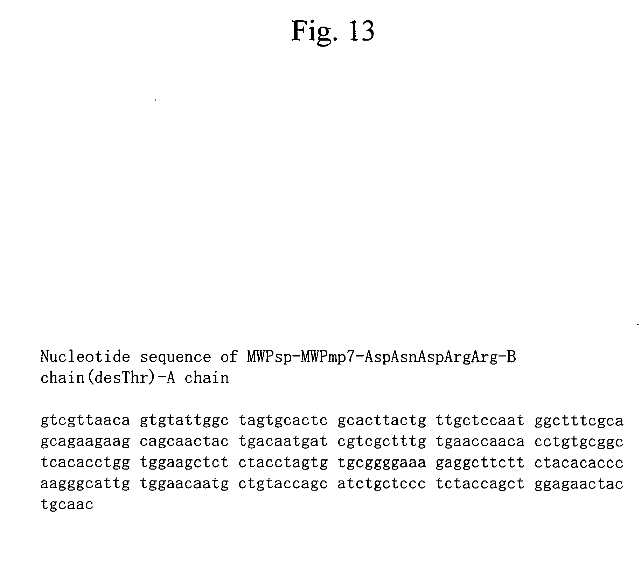

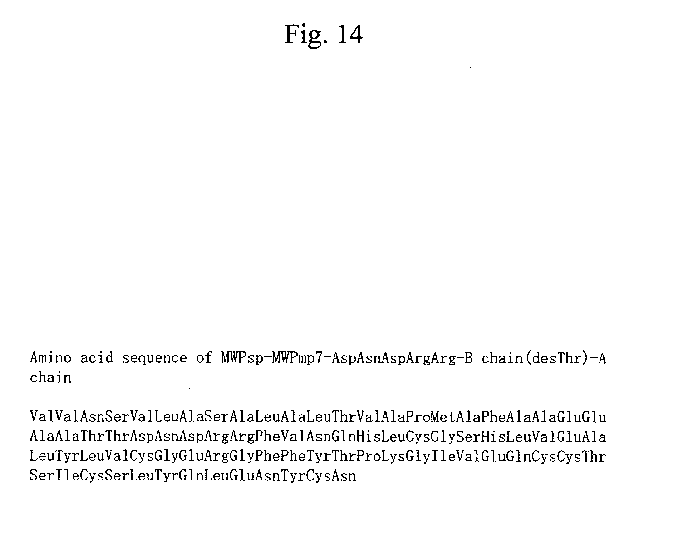

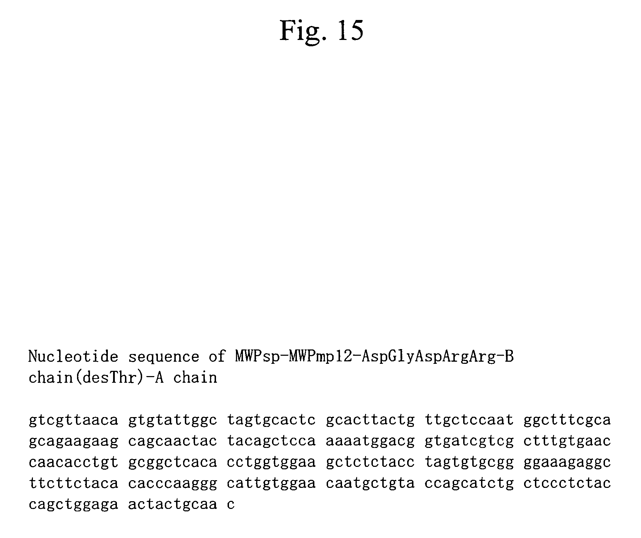

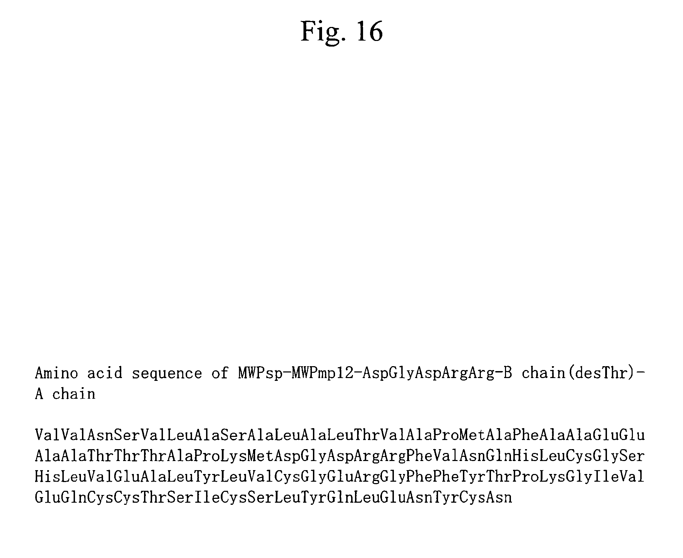

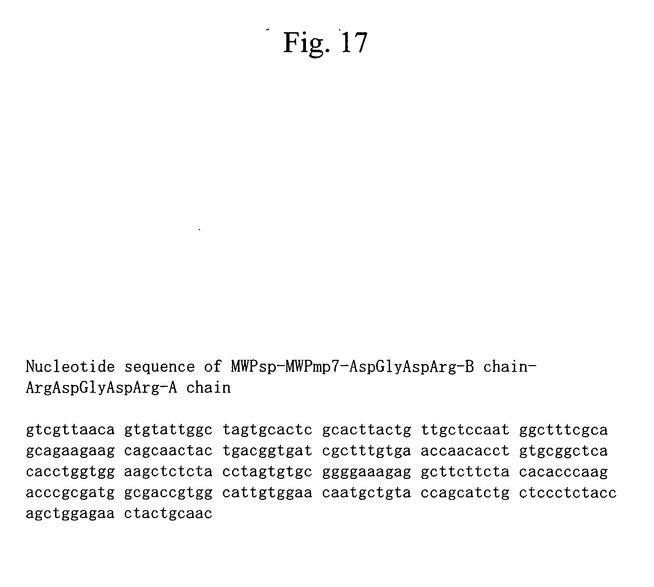

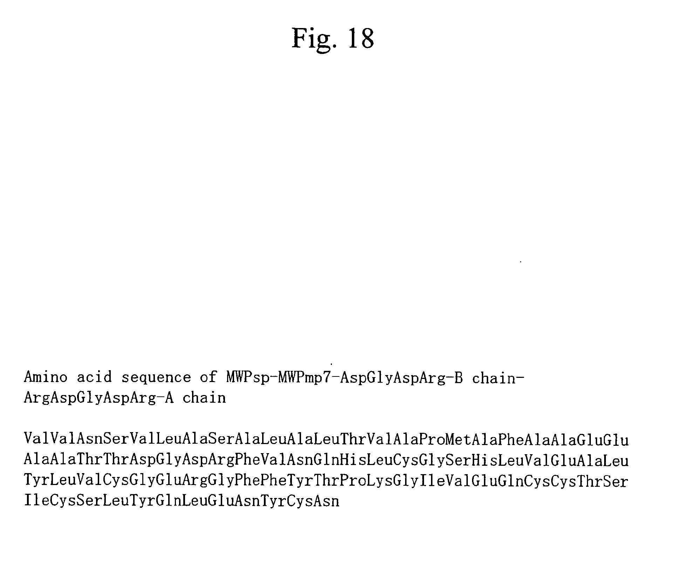

In the same manner as in 2 (1), formation of desired fusion DNA was confirmed. Figs. 1 to 18 shows the amino acid sequences encoded and the DNA nucleotide sequences of the above fusion DNAs.

### 4. Preparation of fusion DNA comprising fusion DNA of 3. above with MWPsp*-hPDI*

A vector incorporating the following fusion DNA was obtained in accordance with the procedure as described in 1 (1), except for the following respects.

Adequate amounts of each of the DNA fragments obtained in 3 (1) to (9) and the DNA fragment MWPsp*-hPDI* as disclosed in WO 01/068884 were mixed, the reaction was carried out at 16°C for 30 minutes using the DNA Ligation Kit (Takara Shuzo Co., Ltd.), and the resultants were used as template DNAs.

The reverse primer 5'-TTACAGTTCATCTTTCACAGCTTTCTG-3' (SEQ ID NO: 50) was used.

A PCR cycle of denaturation at 94°C for 1 minute, annealing at 55°C for 1 minute, and extension of the DNA strand at 72°C for 2 minutes and 30 seconds was repeated 25 times.

In the same manner as in 2 (1), formation of desired fusion DNA was confirmed.

Thus, pMINIPINS∼hPDI* vectors into which a variety of fusion DNAs had been incorporated were obtained. These vectors carry DNAs encoding PDIs, in addition to the fusion DNA (MINIPINS) obtained in 3. above. The Shine-Dalgarno (SD) sequence (i.e., the ribosome binding site) is added to the 5' side of MWPsp of the above DNA fragment MWPsp*-hPDI*. Fusion DNAs each encoding MINIPINS and PDI are excised from the pMINIPINS∼hPDI* vectors, the fusion DNAs are incorporated into adequate expression vectors, and the host cells are transformed with the resulted expression vectors. In the host cells into which such expression vectors had been introduced, fusion DNA of a fusion protein of interest and PDI, that has been inserted into a site downstream of a promoter in an expression vector, is deduced to be transcribed as a single mRNA. Subsequently, the two genes are translated due to the function of SD sequences located on the 5' side of each of the fusion protein and PDI, which would consequently result in coexpression of the fusion protein of interest and PDI in the host cells.

The structures of the fusion proteins encoded by the resulting fusion DNA (MINIPINS) are shown below:
001: MWPsp-MWPmp5-AspGlyAspArgArg-B chain(desThr)-A chain (SEQ ID NO: 19);
002: MWPsp-MWPmp6-AspGlyAspArgArg-B chain(desThr)-A chain (SEQ ID NO: 20);
003: MWPsp-MWPmp6-LeuAsnSerAlaArg-B chain(desThr)-A chain (SEQ ID NO: 21);
004: MWPsp-MWPmp6-GlySerProArg-B chain(desThr)-A chain (SEQ ID NO: 22);
005: MWPsp-MWPmp7-AspGlyAspArgArg-B chain(desThr)-A chain (SEQ ID NO: 23);
006: MWPsp-MWPmp7-AspLeuAspArgArg-B chain(desThr)-A chain (SEQ ID NO: 24);
007: MWPsp-MWPmp7-AspAsnAspArgArg-B chain(desThr)-A chain (SEQ ID NO: 25);
008: MWPsp-MWPmp12-AspGlyAspArgArg-B chain(desThr)-A chain (SEQ ID NO: 26); and
009: MWPsp-MWPmp7-AspGlyAspArg-B chain-ArgAspGlyAspArg-A chain (SEQ ID NO: 27).

In Example II below, various fusion proteins having other structures were subjected to the expression experiment as comparative examples, in addition to Nos. 001 to 009 shown above. Fusion DNAs encoding such other fusion proteins were prepared in the same manner as described above.

Examples of other fusion proteins include the following:
000: MWPmp9-GlySerLeuGlnProArg-B chain-ArgGlyHisArgPro-C peptide-ProArg- A chain;
010: MWPsp-MWPmp6-GluLeuLeuArg-B chain(desThr)-A chain;
011: MWPsp-MWPmp7-AspGlyAspArgArg-B chain-ArgAspGlyAspArg-A chain; and
012: MWPsp-MWPmp0-B chain(desThr)-A chain.

### Example II

### Expression and secretion of fusion DNA

### 1. Measurement of expression level of fusion protein

The fusion protein encoded by the fusion DNA obtained in Example I was expressed. Fig. 19 shows a method for incorporating fusion DNA into the expression vector.

Specifically, the pMINIPINS∼hPDI* vectors into which the above fusion DNA had been introduced were digested with ApaLI and HindIII restriction enzymes, the products were electrophoresed on 0.8% agarose gel, and DNA fragments comprising fusion DNAs were then excised from the gel. Adequate amounts of the excised fusion DNAs and the pNU211R2L5 expression vector for *Brevibacillus brevis* (JP Patent Publication (kokai) No. 7-170984 A (1995)) that has been digested with ApaLI and HindIII were mixed, and the reaction was carried out at 16°C for 30 minutes using the DNA Ligation Kit (Takara Shuzo Co., Ltd.) to incorporate the fusion DNAs into the expression vectors. Thus, expression vectors, pNU-MINIPINS∼hPDI*, into which relevant fusion DNAs had been incorporated, were obtained. The *Brevibacillus brevis* strain 47-5 (FERM BP-1664) was transformed with these expression vectors in accordance with a known technique (Methods in Enzymol., 217: 23-33, 1993), and it was subjected to plate culture on T2 agar plate (polypepton (1%), meat extract (0.5%), yeast extract (0.2%), uracil (0.1 mg/ml), glucose, (1%), erythromycin (10 µg/ml), and agar (1.5%) (pH 7)) to obtain transformants.

The transformants were cultured in T2 medium (the same composition as T2 agar plate except for the absence of agar) at 37°C for 1 day, plasmid DNA was then purified in accordance with a known technique (Molecular Cloning, A Laboratory. Manual, 2nd ed., Cold Spring Harbor Laboratory, 1989), and the plasmid DNA was digested with ApaLI and HindIII to confirm that the fusion DNA of interest was incorporated. Regarding the transformants that were confirmed to comprise the fusion DNA incorporated therein, expression and secretion of fusion proteins encoded by such fusion DNA were tested. Specifically, 50 µl of the cell suspension that had been cultured in T2 medium at 37°C for 1 day was added to 50 ml of M-5YC medium (polypepton (2.05%), yeast extract (0.27%), glucose (3%), MgSO₄·7H₂O (0.009%), MnSO₄·4H₂O (0.0009%), uracil (27 µg/ml), erythromycin (10 µg/ml), (pH 8)), and the resultant was subjected to shaking culture at 30°C for 4 days in a 500-ml conical flask.

After culturing, 1 ml of medium was introduced into a 1.5-ml microtube, and centrifugation was carried out at 15,000 rpm for 20 minutes. To 194 µl of the resulting culture supernatant, 6 µl of 6N hydrochloric acid was added, and the resultant was filtered through a membrane filter (Millipore, pore size 0.22 µm, Cat. No. SLGVR04NL). The filtrate (100 µl) was applied to HPLC (Waters LC-Module 1) in an HPLC column (Waters, Symmetry300™C₁₈, 5 µm, 4.6 × 250 mm). The expression levels of fusion proteins were then determined. Elution and detection were carried out under the following conditions.
Solution A: aqueous solution of 0.1% TFA
Solution B: acetonitrile comprising 0.1% TFA

| Time (min) | Flow | %A | %B |
|---|---|---|---|
| Initial | 0.8 | 72 | 28 |
| 5.00 | 0.8 | 72 | 28 |
| 30.00 | 0.8 | 64 | 36 |
| 32.00 | 0.8 | 64 | 36 |
| 33.00 | 0.8 | 10 | 90 |

Temp: 35°C
SPRG: 30 ml/min
Wave length: 220 nm.

A solution of proinsulin (Sigma, Cat. No. P-4672) dissolved in 0.001N hydrochloric acid (1 mg/ml) was subjected to HPLC under the same conditions. A calibration curve was prepared, and relative amounts of the fusion proteins were determined based thereon.

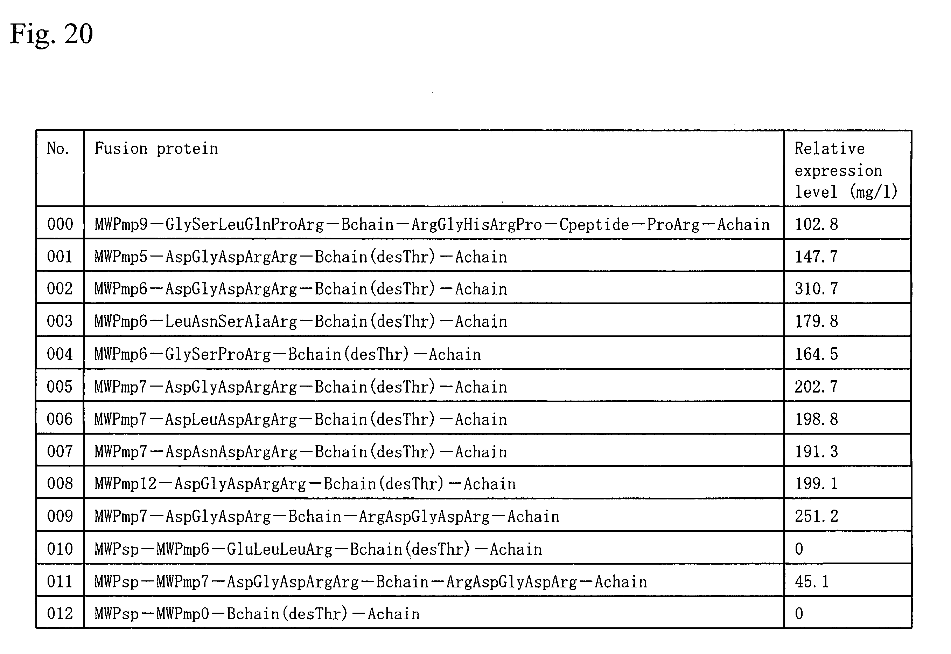

Fig. 20 shows relative expression levels of fusion proteins. In comparison with the expression levels of the fusion protein comprising the mutant proinsulin sequence disclosed in WO 01/068884, MWPmp9-GlySerLeuGlnProArg-B chain-ArgGlyHisArgPro-C peptide-ProArg-A chain (No. 000) (i.e., 102.8 mg/l), the expression levels of fusion proteins having the insulin sequences of the structures shown in Nos. 001 to 009 above were higher by 1.5 to 3 times. The results for Nos. 001 to 009 were also significantly higher than those for fusion proteins of other structures (No. 010 to 012).

### Example III

### Conversion into insulin

### (1) Separation and purification of fusion protein of MWPmp6-AspGlyAspArgArg-B chain(desThr)-A chain

pMINIPINS-transformed bacteria were cultured at 37°C for 1 day, 1.1 ml of the cell suspension was added to 1.1 l of medium (polypepton (3%), yeast extract (0.4%), glucose (3%), MgSO₄·7H₂O (0.01%), MnSO₄·4H₂O (0.001%), erythromycin (10 µg/ml), pH 8), and the resultant was cultured in ajar fermenter at 30°C and 200 rpm with aeration at 1.1 vvm for 4 days. The medium was centrifuged at 9,500 rpm for 20 minutes, the pH level of the obtained supernatant was adjusted to 3 with the use of HCl, and the resultant was allowed to stand at 4°C for 1 hour. The resultant was centrifuged again at 9,500 rpm for 20 minutes, the obtained supernatant was applied to a cation exchange resin equilibrated with 50 mM acetic acid, and fusion proteins were eluted with 50 mM sodium acetate (pH 5.5). Thereafter, the pH level was adjusted to 7.5 with the aid of NaOH. The eluate was then concentrated with exchanging buffers with 20 mM Tris-HCl (pH 8).

### (2) Conversion of fusion protein into insulin

The concentrated fusion proteins were treated in trypsin-containing 50 mM Tris-HCl (pH 8) at 12°C overnight to convert the fusion proteins into des-Thr insulin (100 mg of fusion proteins, 2.4 mg TPCK trypsin/440 ml of the solution). The reaction was terminated with the addition of 10% TFA, the reaction solution was applied to a resin (MCI GEL CHP2MGY, Mitsubishi Chemicals Japan) equilibrated with 18% acetonitrile/0.05% formic acid, and des-Thr insulin was eluted with the aid of 27% acetonitrile/0.05% formic acid. Thereafter, the product was dried in an evaporator.

Subsequently, des-Thr insulin was treated in the reaction solution (10 mM des-Thr insulin, 0.8M Thr-OBut-HCl, 50% DMF:ethanol (1:1), 20 µM trypsin, pH was adjusted to 6.1 with aqueous ammonia) at 15°C for 8 hours to add Thr to des-Thr insulin. Thus, insulin ester was produced. The reaction was terminated with TFA, the reaction solution was applied to a resin (MCI GEL CHP55Y, Mitsubishi Chemicals Japan) equilibrated with 22.5% acetonitrile/0.05% formic acid, and insulin ester was eluted with the use of 29.3% acetonitrile/0.05% formic acid.

Finally, the eluted insulin ester was dried and treated with anisole:TFA (1:9) to obtain insulin.

### (3) Peptide mapping of des-Thr insulin

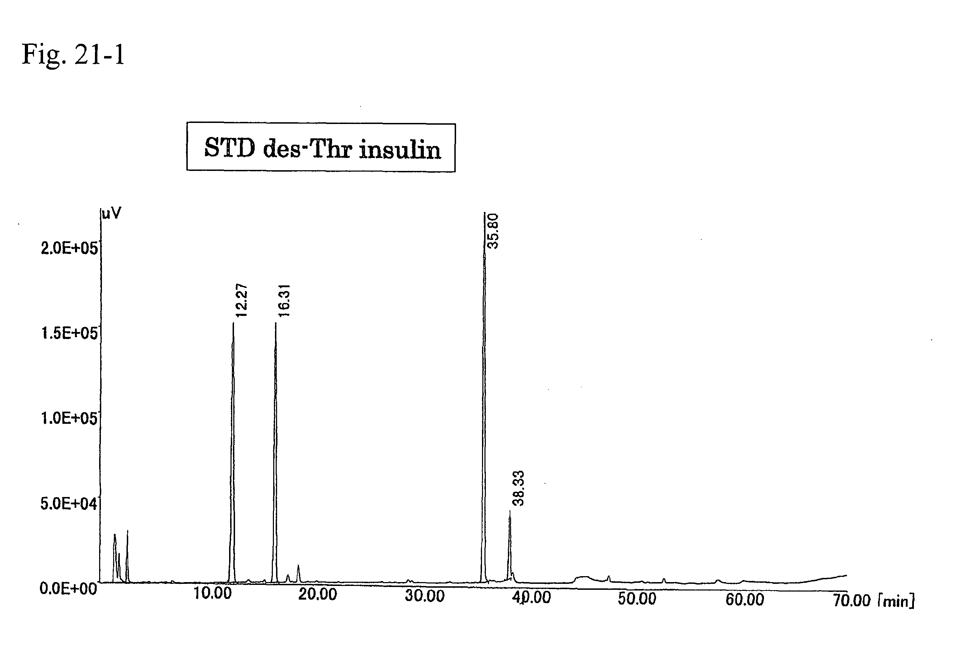

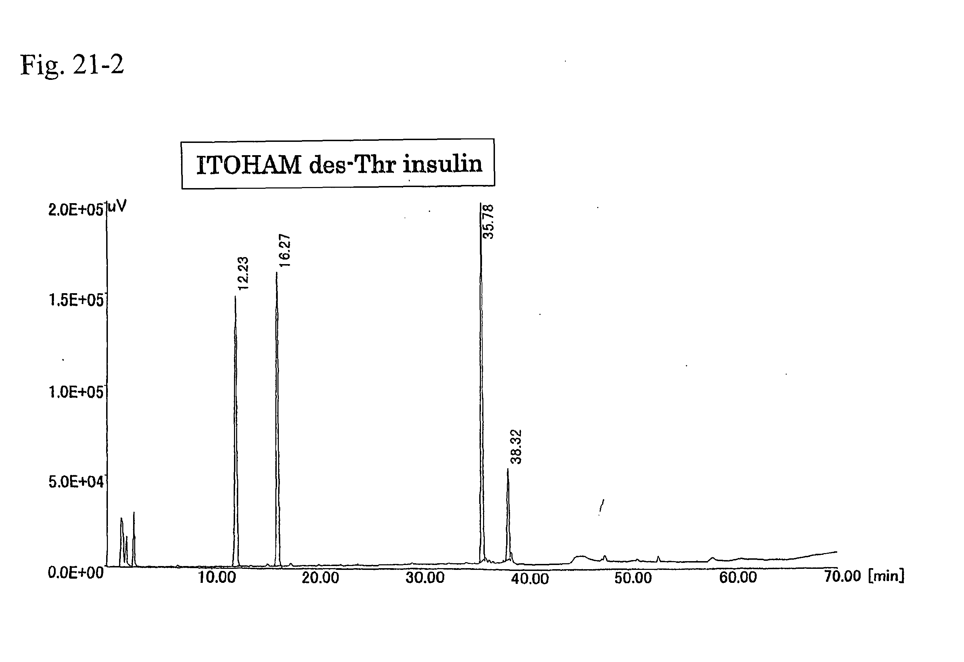

Commercialized insulin (Intergen) was treated with carboxypeptidase A (25°C, 1 hour, 0.2N NH₄HCO₃, pH 8.4, carboxypeptidase A (0.3 U/mg insulin)) to obtain des-Thr insulin (hereafter referred to as "STD des-Thr insulin"). The des-Thr insulin obtained in (2) above (hereafter referred to as "ITOHAM des-Thr insulin") and STD des-Thr insulin (5 nmole each) were dissolved in 50 µl of a solution of 0.1M ammonium bicarbonate and 2 mM EDTA (pH 7.8), 1.35 ml of an aqueous solution of V8 protease (2 µg/ml, Wako Pure Chemical Industries, Ltd.) was added, and the reaction was allowed to proceed at 25°C for 24 hours. Subsequently, pH was adjusted to 2 with the addition of 1% TFA to terminate the reaction. The reaction solution was then applied to Vydac218TP54 (4.6 × 250 mm, C₁₈ column), equilibrated with a solution of 5% acetonitrile and 0.1% TFA, and then eluted with a gradient from a solution of 5% acetonitrile and 0.1% TFA to a solution of 35% acetonitrile and 0.1% TFA. Fig. 21 shows the elution patterns. The elution pattern of ITOHAM des-Thr insulin was similar to that of STD des-Thr insulin. It was thus concluded that the modes of disulfide linkage formation of these des-Thr insulins were comparable to each other.

### (4) HPLC elution patterns of insulin

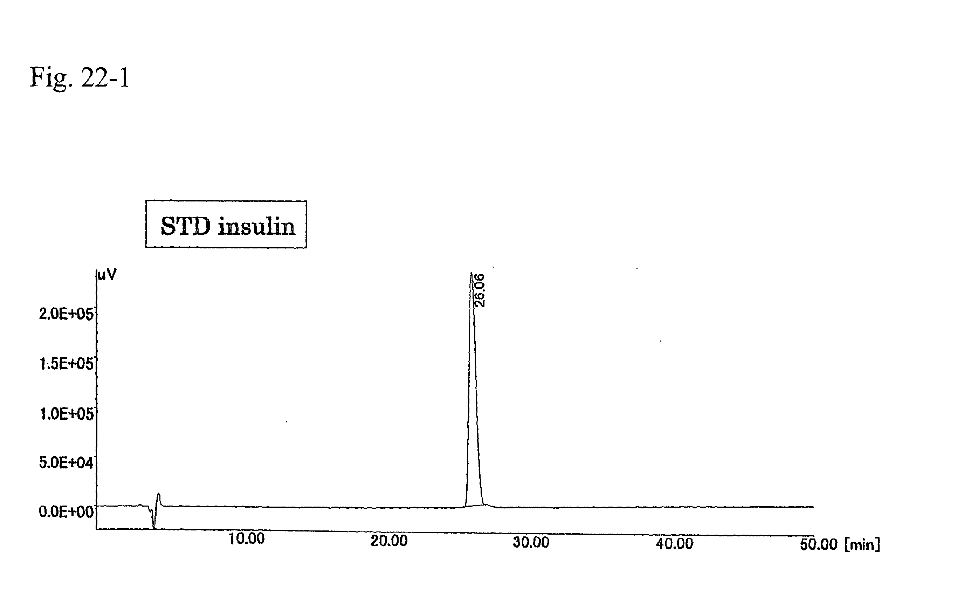

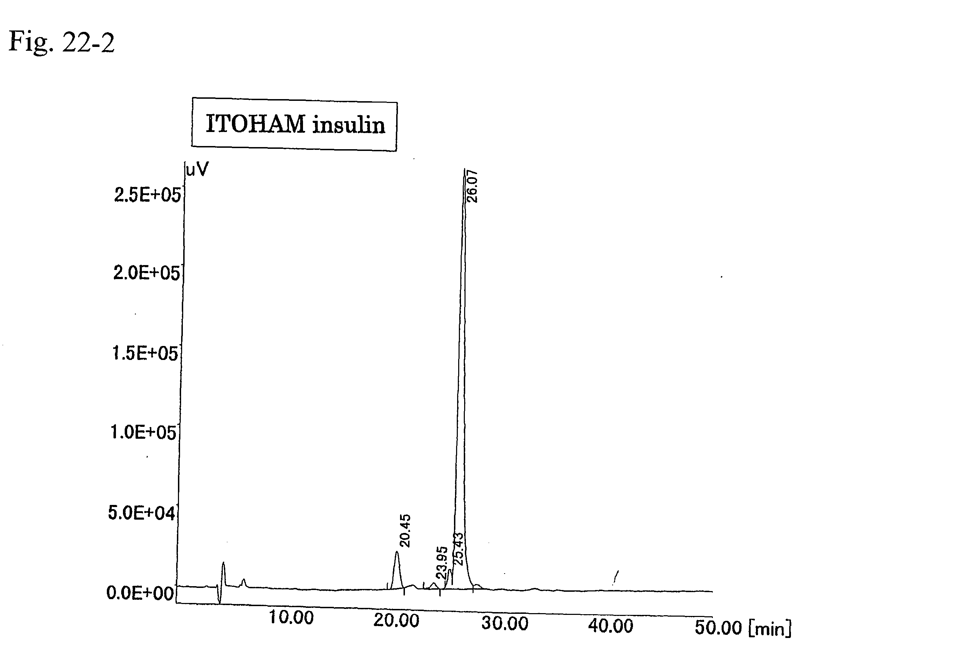

The insulin obtained in (3) above (hereafter referred to as "ITOHAM insulin") and the commercialized insulin (Intergen, hereafter referred to as "STD insulin") were applied to the YMC C₄ column (4.6 × 250 mm) and then eluted with a gradient of 25% to 35% acetonitrile/0.1% TFA. As shown in Fig. 22, the elution patterns of both insulins were identical to each other.

This demonstrates that insulins having adequate disulfide linkages could be formed from one of the fusion proteins that were expressed at high levels in the *Brevibacillus brevis* expression system, i.e., MWPmp6-AspGlyAspArgArg-B chain(desThr)-A chain.

### Industrial Applicability

The present invention provides a method for producing functional insulins that can be used for treatment of diseases such as diabetes mellitus with industrially acceptable efficiency.

## Claims

1. DNA encoding a fusion protein comprising: a signal peptide from MWP, which is a cell-wall protein (CWP) of a bacterium of the genus *Bacillus* or *Brevibacillus;* a leader peptide comprising 5 to 7 or 12 amino acid residues from CWP of a bacterium of the genus *Bacillus* or *Brevibacillus;* a linker peptide comprising an amino acid sequence represented by the general formula: (Asp, Leu, or Gly)(Gly, Asn, Ser, or Leu)(Asp, Ser, or Pro)(Arg, Ala, or none)Arg (SEQ ID NO: 51 or 52); and an amino acid sequence of an insulin precursor, ligated in the order.

2. DNA according to claim 1, wherein the linker peptide comprises an amino acid sequence as shown in any one of SEQ ID NOs: 1 to 6.

3. DNA according to claim 1 or 2, wherein the leader peptide is from MWP.

4. DNA according to any one of claims 1 to 3, wherein the insulin precursor comprises an amino acid sequence as shown in SEQ ID NO: 8 or 9.

5. DNA according to any one of claims 1 to 4, wherein the fusion protein comprises an amino acid sequence as shown in any one of SEQ ID NOs: 10 to 18.

6. DNA according to any one of claims 1 to 5, which comprises a nucleotide sequence as shown in any one of SEQ ID NOs: 19 to 27.

7. A vector comprising DNA according to any one of claims 1 to 6.

8. The vector according to claim 7, wherein the DNA is operably linked to a site downstream of a promoter sequence from a bacterium.

9. The vector according to claim 8, wherein the promoter is from a bacterium of the genus *Bacillus* or *Brevibacillus.*

10. The vector according to any one of claims 7 to 9, which further comprises DNA encoding a protein disulfide isomerase (PDI).

11. A host cell comprising the vector according to any one of claims 7 to 10.

12. The host cell according to claim 11, which is a bacterium of the genus *Bacillus* or *Brevibacillus.*

13. The host cell according to claim 12, wherein the bacterium is *Brevibacillus brevis.*

14. A method for producing insulin comprising steps of: culturing the host cell according to any one of claims 11 to 13; expressing a desired fusion protein from the host cell; and recovering the expressed polypeptide from the cell or medium.

15. The method according to claim 14, which further comprises a step of enzymatically treating the recovered polypeptide.

16. The method according to claim 15, wherein the enzymatic treatment is treatment with trypsin.

17. The method according to any one of claims 14 to 16, wherein the polypeptide is recovered from the medium.

18. A fusion protein having an amino acid sequence as shown in any one of SEQ ID NOs: 10 to 18.
